# EUROPÄISCHE PATENTSCHRIFT

(11) **EP 2 590 987 B1**
(45) Veröffentlichungstag und Bekanntmachung des Hinweises auf die Patenterteilung: **09.03.2016**
(21) Anmeldenummer: 11745710.1
(22) Anmeldetag: 05.07.2011
(51) Int. Cl.: C07D 519/00, A61K 31/437, A61P 9/00

(54) **ANNELLIERTE 4-AMINOPYRIMIDINE UND IHRE VERWENDUNG ALS STIMULATOREN DER LÖSLICHEN GUANYLATCYCLASE**
CONDENSED 4-AMINOPYRIMIDINES AND THEIR USE AS STIMULATORS OF SOLUBLE GUANYLATE CYCLASE
DÉRIVÉS DE 4-AMINOPYRIMIDINE CONDENSÉES ET LEUR UTILISATION EN TANT QUE STIMULATEURS DE LA GUANYLATE CYCLASE SOLUBLE

(30) Priorität: 21.04.2011 DE 102011007891; 09.07.2010 DE 102010031148
(43) Veröffentlichungstag der Anmeldung: 15.05.2013
(73) Patentinhaber: Bayer Intellectual Property GmbH, 40789 Monheim (DE)
(72) Erfinder: FOLLMANN, Markus, 50859 Köln (DE); STASCH, Johannes-Peter, 42651 Solingen (DE); REDLICH, Gorden, 44799 Bochum (DE); ACKERSTAFF, Jens, 10437 Berlin (DE); GRIEBENOW, Nils, 41541 Dormagen (DE); KNORR, Andreas, 40699 Erkrath (DE); WUNDER, Frank, 42117 Wuppertal (DE); LI, Volkhart Min-Jian, 42553 Velbert (DE)
(86) Internationale Anmeldenummer: PCT/EP2011/061306
(87) Internationale Veröffentlichungsnummer: WO 2012/004259

(56) Entgegenhaltungen:
- WO-A1-2010/065275

## Beschreibung

Die vorliegende Anmeldung betrifft neue annellierte 4-Aminopyrimidine, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Arteriosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol [YC-1; Wu et al., Blood 84 (1994), 4226; Mülsch et al., Brit. J. Pharmacol. 120 (1997), 681], Fettsäuren [Goldberg et al., J. Biol. Com. 252 (1977), 1279], Diphenyliodonium-hexafluorophosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223).

Als Stimulatoren der löslichen Guanylatcyclase werden in WO 00/06569 annellierte Pyrazol-Derivate und in WO 03/095451 Carbamat-substitutierte 3-Pyrimidinyl-Pyrazolopyridine offenbart. WO 2010/065275 offenbart substituierte Pyrrolo- und Dihydropyridopyrimidine als sGC Aktivatoren.

Aufgabe der vorliegenden Erfindung war die Bereitstellung neuer Substanzen, die als sehr potente Stimulatoren der löslichen Guanylatcyclase wirken.

Gegenstand der vorliegenden Erfindung sind Verbindungen der allgemeinen Formel (I) 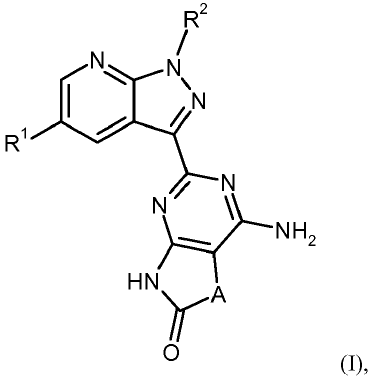 in welcher
- A: für (C₁-C₃)-Alkandiyl oder eine Gruppe der Formel 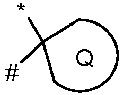 steht, wobei
* für die Anknüpfstelle an den Pyrimidinring steht,
# für die Anknüpfstelle an die Carbonylgruppe steht,
der Ring Q für einen 4- bis 6-gliedrigen Heterocyclus steht,
   und
wobei (C₁-C₃)-Alkandiyl mit 1 oder 2 Substituenten unabhängig voneinander ausgewählt aus der Gruppe Fluor, Trifluormethyl, (C₁-C₄)-Alkyl, Hydroxy und Amino substituiert sein kann,
   worin (C₁-C₄)-Alkyl mit 1 bis 3 Substituenten unabhängig voneinander ausgewählt aus der Gruppe Fluor, Trifluormethyl und Hydroxy substituiert sein kann,
- R¹: für Wasserstoff oder Fluor steht,
- R²: für Benzyl steht,
wobei Benzyl mit 1 bis 3 Substituenten Fluor substituiert ist,
sowie ihre *N*-Oxide, Salze, Solvate, Salze der *N*-Oxide und Solvate der *N*-Oxide und Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Erfindungsgemäße Verbindungen sind ebenso *N*-Oxide der Verbindungen der Formel (I) sowie deren Salze, Solvate und Solvate der Salze.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Trisethanolamin, Dicyclohexylamin, Dimethylaminoethanol, Prokain, Dibenzylamin, *N*-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ¹⁴C, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

In der Formel der Gruppe, für die A stehen kann, steht der Endpunkt der Linie, an dem das Zeichen * bzw. # steht, nicht für ein Kohlenstoffatom beziehungsweise eine CH₂-Gruppe, sondern ist Bestandteil der Bindung zu dem jeweils bezeichneten Atom, an das A gebunden ist.

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit 1 bis 4 Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl.
Alkandiyl steht im Rahmen der Erfindung für einen linearen divalenten Alkylrest mit 1 bis 3 Kohlenstoffatomen. Beispielhaft seien genannt: Methylen, Ethan-1,2-diyl oder Propan-1,3-diyl.
Heterocyclus steht im Rahmen der Erfindung für einen gesättigten Heterocyclus mit insgesamt 4 bis 6 Ringatomen, der ein oder zwei Ring-Heteroatome aus der Reihe N, O und/oder S enthält und über ein Ring-Kohlenstoffatom verknüpft ist. Beispielhaft seien genannt: Azetidinyl, Oxetanyl, Pyrrolidinyl, Pyrazolidinyl, Tetrahydrofuranyl, Piperidinyl, Piperazinyl, Tetrahydropyranyl, Morpholinyl und Thiomorpholinyl. Bevorzugt sind Azetidinyl, Oxetanyl, Pyrrolidinyl, Tetrahydrofuranyl, Piperidinyl und Tetrahydropyranyl.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Bervorzugt sind im Rahmen der vorliegenden Erfindung Verbindungen der allgemeinen Formel (I-1) 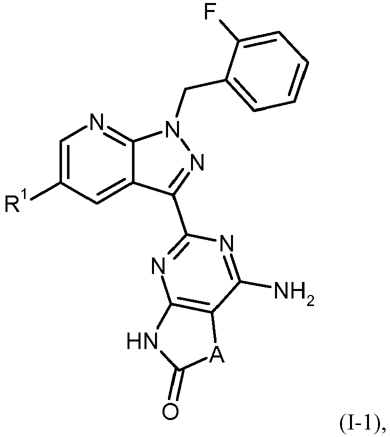 in welcher
- A: für (C₁-C₃)-Alkandiyl oder eine Gruppe der Formel 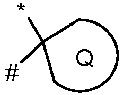 steht, wobei
* für die Anknüpfstelle an den Pyrimidinring steht,
# für die Anknüpfstelle an die Carbonylgruppe steht,
der Ring Q für einen 4- bis 6-gliedrigen Heterocyclus steht,
   und
   wobei (C₁-C₃)-Alkandiyl mit 1 oder 2 Substituenten unabhängig voneinander ausgewählt aus der Gruppe Fluor, Trifluormethyl und (C₁-C₄)-Alkyl substituiert sein kann,
- R¹: für Wasserstoff oder Fluor steht,
sowie ihre *N*-Oxide, Salze, Solvate, Salze der *N*-Oxide und Solvate der *N*-Oxide und Salze.

Bevorzugt sind im Rahmen der vorliegenden Erfindung auch Verbindungen der Formel (I), in welcher
- A: für Gruppe der Formel 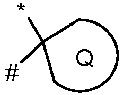 steht, wobei
* für die Anknüpfstelle an den Pyrimidinring steht,
# für die Anknüpfstelle an die Carbonylgruppe steht,
der Ring Q für einen Azetidinyl-, Oxetanyl-, Pyrrolidinyl-, Tetrahydrofuranyl-, Piperidinyl- oder Tetrahydropyranylring steht,
- R¹: für Wasserstoff oder Fluor steht,
- R²: für

Benzyl steht,
wobei Benzyl mit 1 bis 3 Substituenten Fluor substituiert ist,
sowie ihre Salze, Solvate und Solvate der Salze.

Bevorzugt sind im Rahmen der vorliegenden Erfindung auch Verbindungen der Formel (I), in welcher
- A: für (C₁-C₃)-Alkandiyl
wobei (C₁-C₃)-Alkandiyl mit 1 oder 2 Substituenten unabhängig voneinander ausgewählt aus der Gruppe Fluor, (C₁-C₄)-Alkyl, Hydroxy und Amino substituiert sein kann,
und
wobei (C₁-C₃)-Alkandiyl mit 1 Substituenten ausgewählt aus der Gruppe Fluor und Trifluormethyl substituiert ist,
- R¹: für Wasserstoff oder Fluor steht,
- R²: für
Benzyl steht,
wobei Benzyl mit 1 bis 3 Substituenten Fluor substituiert ist,
sowie ihre Salze, Solvate und Solvate der Salze.

Bevorzugt sind im Rahmen der vorliegenden Erfindung auch Verbindungen der Formel (I-1), in welcher
- A: für Gruppe der Formel 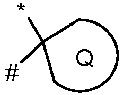 steht, wobei
* für die Anknüpfstelle an den Pyrimidinring steht,
# für die Anknüpfstelle an die Carbonylgruppe steht,
der Ring Q für einen Azetidinyl-, Oxetanyl-, Pyrrolidinyl-, Tetrahydrofuranyl-, Piperidinyl- oder Tetrahydropyranylring steht,
- R¹: für Wasserstoff oder Fluor steht,
sowie ihre Salze, Solvate und Solvate der Salze.

Bevorzugt sind im Rahmen der vorliegenden Erfindung auch Verbindungen der Formel (I-1), in welcher
- A: für (C₁-C₃)-Alkandiyl
wobei (C₁-C₃)-Alkandiyl mit 1 oder 2 Substituenten unabhängig voneinander ausgewählt aus der Gruppe Fluor und (C₁-C₄)-Alkyl substituiert sein kann,
und
wobei (C₁-C₃)-Alkandiyl mit 1 Substituenten ausgewählt aus der Gruppe Fluor und Trifluormethyl substituiert ist,
- R¹: für Wasserstoff oder Fluor steht,
sowie ihre Salze, Solvate und Solvate der Salze.

Bevorzugt sind im Rahmen der vorliegenden Erfindung auch Verbindungen der Formel (I), in welcher
- A: für Methylen oder Ethan-1,2-diyl steht,
wobei Methylen und Ethan-1,2-diyl mit 1 oder 2 Substituenten unabhängig voneinander ausgewählt aus der Gruppe Fluor und Trifluormethyl substituiert sind,
sowie ihre Salze, Solvate und Solvate der Salze.

Bevorzugt sind im Rahmen der vorliegenden Erfindung auch Verbindungen der Formel (I), in welcher
- A: für Gruppe der Formel 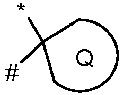 steht, wobei
* für die Anknüpfstelle an den Pyrimidinring steht,
# für die Anknüpfstelle an die Carbonylgruppe steht,
der Ring Q für einen 4- bis 6-gliedrigen Heterocyclus steht,
sowie ihre Salze, Solvate und Solvate der Salze.

Bevorzugt sind im Rahmen der vorliegenden Erfindung auch Verbindungen der Formel (I), in welcher
- A: für Gruppe der Formel 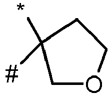 steht, wobei
* für die Anknüpfstelle an den Pyrimidinring steht,
# für die Anknüpfstelle an die Carbonylgruppe steht,
sowie ihre Salze, Solvate und Solvate der Salze.

Bevorzugt sind im Rahmen der vorliegenden Erfindung auch Verbindungen der Formel (I), in welcher R¹ für Fluor steht,
sowie ihre Salze, Solvate und Solvate der Salze.

Bevorzugt sind im Rahmen der vorliegenden Erfindung auch Verbindungen der Formel (I), in welcher
- R²: für 3,3,4,4,4-Pentafluorbut-lyl steht,
sowie ihre Salze, Solvate und Solvate der Salze.

Bevorzugt sind im Rahmen der vorliegenden Erfindung auch Verbindungen der Formel (I), in welcher
- R²: für Benzyl steht,
wobei Benzyl mit 1 bis 3 Substituenten Fluor substituiert ist,
sowie ihre Salze, Solvate und Solvate der Salze.

Besonders bevorzugt im Rahmen der vorliegenden Erfindung sind folgende Verbindungen der Formel (I):
4-Amino-2-[1-(2-fluorbenzyl)-1H-pyrazolo[3,4-b]pyridin-3-yl]-5,7-dihydro-6H-pyrrolo[2,3-d]pyrimidin-6-on
4-Amino-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on
4-Amino-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on
4'-Amino-2'-[1-(2-fluorbenzyl)-1H-pyrazolo[3,4-b]pyridin-3-yl]-4,5-dihydrospiro[furan-3,5'-pyrrolo[2,3-d]pyrimidin]-6'(7'H)-on
4-Amino-2-[1-(2-fluorbenzyl)-1H-pyrazolo[3,4-b]pyridin-3-yl]-5-methyl-5,8-dihydropyrido[2,3-d]pyrimidin-7(6H)-on
4-Amino-2-[1-(2-fluorbenzyl)-1H-pyrazolo[3,4-b]pyridin-3-yl]-5-(trifluormethyl)-5,8-dihydropyrido[2,3-d]pyrimidin-7(6H)-on

Insbesondere bevorzugt im Rahmen der vorliegenden Erfindung sind folgende Verbindungen der Formel (I):
4'-Amino-2'-[1-(2-fluorbenzyl)-1H-pyrazolo[3,4-b]pyridin-3-yl]-4,5-dihydrospiro[furan-3,5'-pyrrolo[2,3-d]pyrimidin]-6'(7'H)-on
4-Amino-2-[1-(2-fluorbenzyl)-1H-pyrazolo[3,4-b]pyridin-3-yl]-5-(trifluormethyl)-5,8-dihydropyrido[2,3-d]pyrimidin-7(6H)-on

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I) dadurch gekennzeichnet, dass man
[A] eine Verbindung der Formel (II) 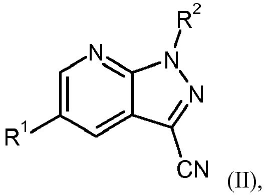 in welcher R¹ und R² jeweils die oben angegebenen Bedeutungen haben,
   unter sauren Bedingungen in eine Verbindung der Formel (III) 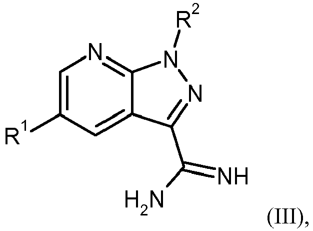 in welcher R¹ und R² jeweils die oben angegebenen Bedeutungen haben,
   überführt, diese im Folgenden in einem inerten Lösungsmittel in Gegenwart einer geeigneten Base mit einer Verbindung der Formel (IV) 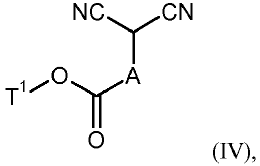 in welcher A die oben angegebene Bedeutung hat und
   T¹ für (C₁-C₄)-Alkyl steht,
   zu einer Verbindung der Formel (I) 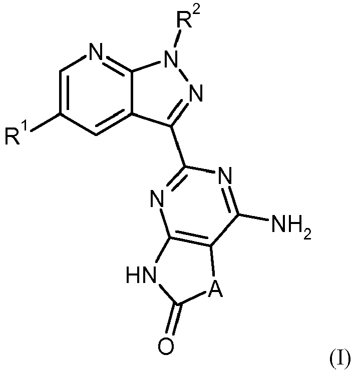 in welcher A, R¹ und R² jeweils die oben angegebenen Bedeutungen haben,
   umsetzt
   oder
[B] eine Verbindung der Formel (III) in einem inerten Lösungsmittel in Gegenwart einer geeigneten Base mit einer Verbindung der Formel (V) 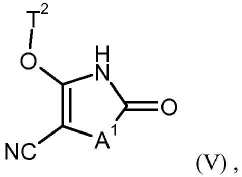 in welcher
   - A¹: für (C₂-C₃)-Alkandiyl steht,
   wobei (C₂-C₃)-Alkandiyl mit 1 oder 2 Substituenten unabhängig voneinander ausgewählt aus der Gruppe Fluor, Trifluormethyl und (C₁-C₄)-Alkyl substituiert sein kann,
   worin (C₁-C₄)-Alkyl mit 1 bis 3 Substituenten unabhängig voneinander ausgewählt aus der Gruppe Fluor, Trifluormethyl und Hydroxy substituiert sein kann,
   und
   T² für (C₁-C₄)-Alkyl steht,
   zu einer Verbindung der Formel (I-A) 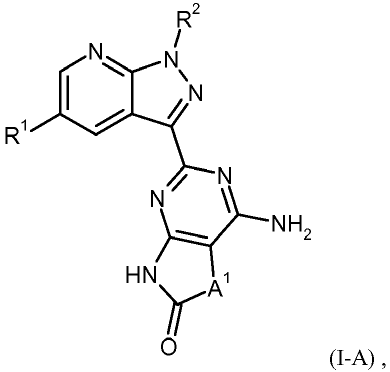 in welcher A¹, R¹ und R² jeweils die oben angegebenen Bedeutungen haben,
   umsetzt,
gegebenenfalls die resultierenden Verbindungen der Formeln (I) und (I-A) gegebenenfalls mit den entsprechenden (i) Lösungsmitteln und/oder (ii) Säuren oder Basen in ihre Solvate, Salze und/oder Solvate der Salze überführt.

Die Umsetzung (II) → (III) erfolgt nach den dem Fachmann bekannten Methoden in einem zweistufigen Prozess zunächst unter Bildung des Iminoesters mit Natriummethanolat in Methanol bei 0°C bis +40°C und anschliessender nucleophiler Addition eines Ammoniak-Äquivalents wie beispielsweise Ammoniak oder Ammoniumchlorid in einer geeigneten Säure unter Bildung des Amidins (III) bei +50 bis +150°C.

Geeignete Säure für die Bildung des Amidins (III) sind anorganische Säuren wie beispielsweise Chlorwasserstoff/ Salzsäure, Schwefelsäure, Polyphosphorsäure oder Phosphorsäure oder organische Säuren wie beispielsweise Essigsäure, Trifluoressigsäure oder Ameisensäure. Bervorzugt werden Salzsäure oder Essigsäure verwendet.

Inerte Lösungsmittel für den Verfahrensschritt (III) + (IV) → (I) sind beispielsweise Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist tert.-Butanol.

Geeignete Basen für den Verfahrensschritt (III) + (IV) → (I) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Kalium-tert.-butylat.

Die Reaktion (III) + (IV) → (I) wird im Allgemeinen in einem Temperaturbereich von +20°C bis +150°C, bevorzugt bei +75°C bis +100°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für den Verfahrensschritt (III) + (V) → (I-A) sind beispielsweise Alkohole wie Methanol, Ethanol, n-Propanol, Isopropanol, n-Butanol oder tert.-Butanol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen.

Geeignete Basen für den Verfahrensschritt (III) + (V) → (I-A) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN).

Bevorzugt wird Natriummethanolat in Methanol oder Kalium-tert.-butylat in tert.-Butanol eingesetzt.

Die Reaktion (III) + (V) → (I-A) wird im Allgemeinen in einem Temperaturbereich von +20°C bis +150°C, bevorzugt bei +60°C bis +100°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die zuvor beschrieben Verfahren werden anhand der folgenden Syntheseschemata (Schema 1 und 2) beispielhaft erläutert:

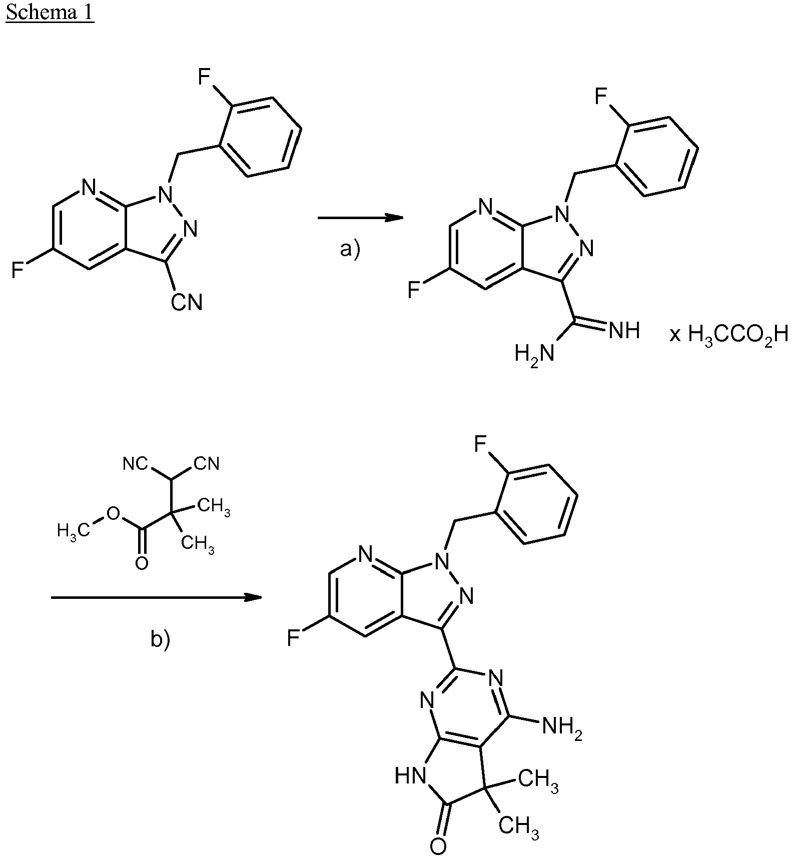

### [a): 1. Natriummethylat, Methanol 2. Ammoniumchlorid, Essigsäure; b): KOt-Bu, t-BuOH].

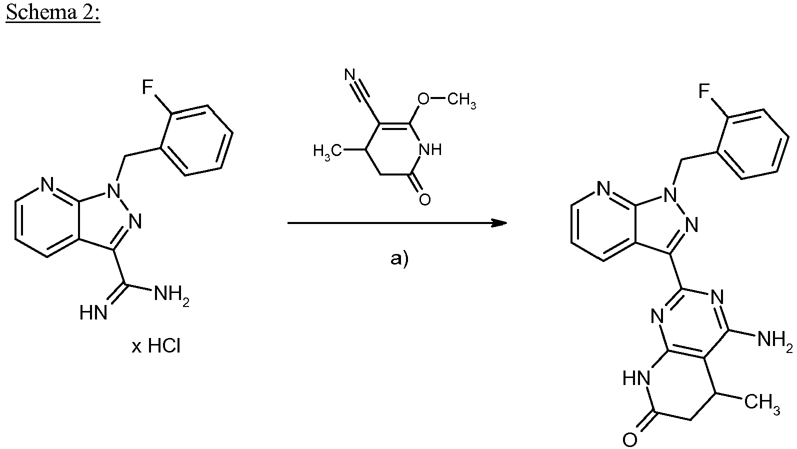

### [a): NaOMe, Methanol, 65°C].

Die Verbindungen der Formel (II) sind literaturbekannt (siehe z.B. WO 03/095451, Beispiel 4A) oder können hergestellt werden, indem man eine Verbindung der Formel (VI) 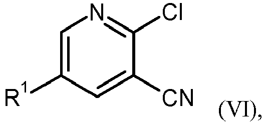 in welcher R¹ die oben angegebene Bedeutung hat,
in einem inerten Lösungsmittel mit Hydrazinhydrat zu einer Verbindung der Formel (VII) 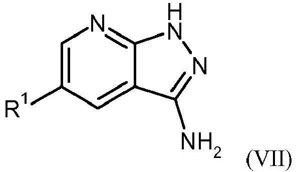 in welcher R¹ die oben angegebene Bedeutung hat,
zyklisiert, diese anschliessend in einem inerten Lösungsmittel in Gegenwart einer geeigneten LewisSäure zunächst mit Isopentylnitrit zum entsprechenden Diazoniumsalz umsetzt, und dieses dann direkt mit Natriumiodid in eine Verbindung der Formel (VIII) 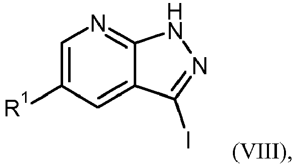 in welcher R¹ die oben angegebene Bedeutung hat,
überführt, diese im Folgenden in einem inerten Lösungsmittel in Gegenwart einer geeigneten Base mit der Verbindung der Formel (IX)

R²-X¹ (IX),

in welcher R² die oben angegebene Bedeutung hat und
- X¹: für eine geeignete Abgangsgruppe, wie beispielsweise Tosylat, Mesylat oder Halogen, insbesondere Brom oder Iod, steht,
zu einer Verbindung der Formel (X) 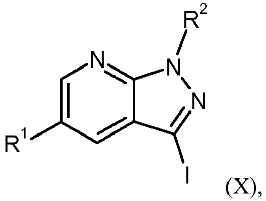 in welcher R¹ und R² jeweils die oben angegebene Bedeutung hat,
umsetzt, und diese anschließend in einem inerten Lösungsmittel mit Kupfercyanid umsetzt.

Inerte Lösungsmittel für den Verfahrensschritt (VI) → (VII) sind Alkohole wie Methanol, Ethanol, n-Propanol, Isopropanol, n-Butanol, tert.-Butanol oder 1,2-Ethandiol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist 1,2-Ethandiol.

Die Reaktion (VI) → (VII) wird im Allgemeinen in einem Temperaturbereich von +60°C bis +200°C, bevorzugt bei +120°C bis +180°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für die Umsetzung (VII) → (VIII) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP), Pyridin oder Acetonitril. Bevorzugt ist DMF.

Als Lewis-Säuren für den Verfahrensschritt (VII) → (VIII) eignen sich Bortrifluorid-Diethylether-Komplex, Cer(IV)ammoniumnitrat (CAN), Zinn(II)chlorid, Lithiumperchlorat, Zink(II)chlorid, Indium(III)chlorid oder Indium(III)bromid. Bevorzugt ist Bortrifluorid-Diethylether-Komplex.

Die Reaktion (VII) → (VIII) wird im Allgemeinen in einem Temperaturbereich von -78°C bis +40°C, bevorzugt bei 0°C bis +20°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für die Umsetzung (VIII) + (IX) → (X) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP), Pyridin, Acetonitril. Bevorzugt ist DMF.

Geeignete Basen für den Verfahrensschritt (VIII) + (IX) → (X) sind Alkalihydride wie Kaliumhydrid oder Natriumhydrid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Cäsiumcarbonat.

Die Reaktion (VIII) + (IX) → (X) wird im Allgemeinen in einem Temperaturbereich von 0°C bis +60°C, bevorzugt bei +10°C bis +25°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für den Verfahrensschritt (X) → (II) sind beispielsweise Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N*,*N*'-Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist DMSO.

Die Reaktion (X) → (II) wird im Allgemeinen in einem Temperaturbereich von +20°C bis +180°C, bevorzugt bei +100°C bis +160°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Das beschriebene Herstellverfahren kann durch das folgende Syntheseschema (Schema 3) beispielhaft verdeutlicht werden:

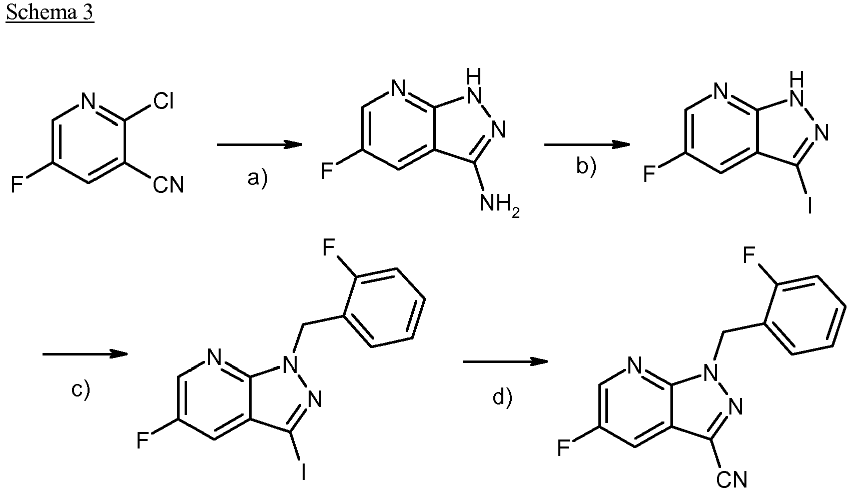

### [a): Hydrazinhydrat, 1,2-Ethandiol; b): iso-Pentylnitrit, NaI, THF; b): 2-Fluorbenzylbromid, Cs₂CO₃, DMF; d): CuCN, DMSO].

Die Verbindung der Formel (VI) ist literaturbekannt [vgl. z.B. Winn M., J. Med. Chem. 1993, 36, 2676-7688; EP 634 413-A1; CN 1613849-A; EP 1626045-A1; WO 2009/018415], kann in Analogie zu literaturbekannten Verfahren oder wie im nachstehenden Syntheseschema gezeigt (Schema 4) hergestellt werden:

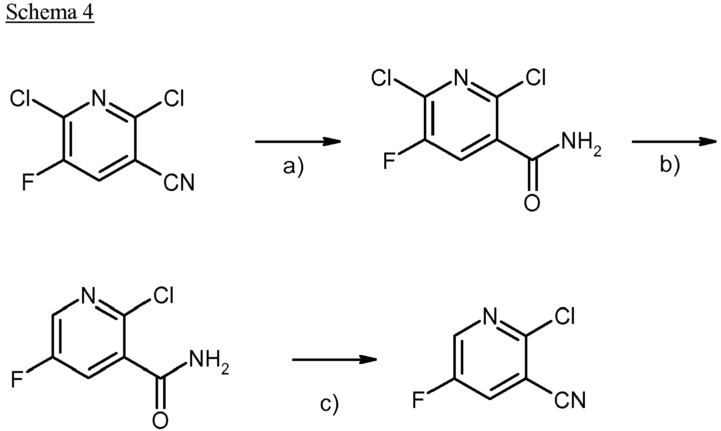

### [a): Schwefelsäure; b): Zink, Methanol, Eisessig; c): Trifluoressigsäureanhydrid, Dichlormethan].

Die Verbindungen der Formeln (IV) und (V) sind kommerziell erhältlich, literaturbekannt, können in Analogie zu literaturbekannten Verfahren oder wie in den nachfolgenden Syntheseschemata (Schemata 5 und 6) beispielhaft gezeigt, hergestellt werden:

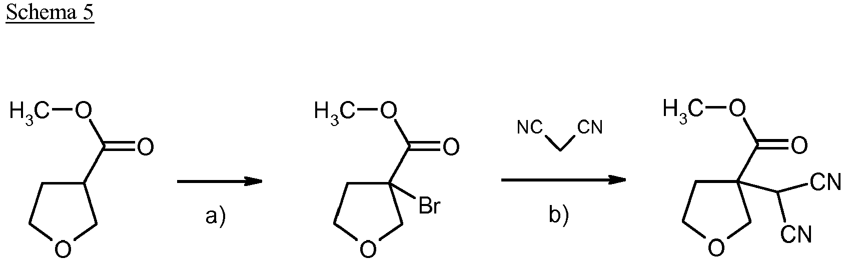

### [a): 1. LiHMDS, -78°C, THF, 2. NBS; b): NaH, 50°C, THF].

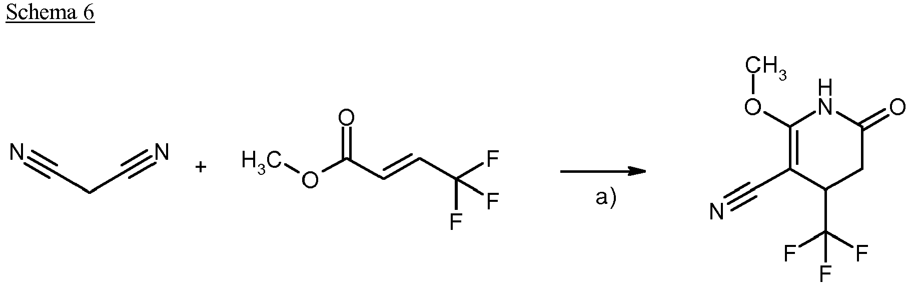

### [a): NaOMe, MeOH, 65°C].

Die erfindungsgemäßen Verbindungen sind potente Stimulatoren der löslichen Guanylatcyclase, besitzen wertvolle pharmakologische Eigenschaften und eignen sich daher zur Behandlung und/ oder Prophylaxe von Erkrankungen bei Menschen und Tieren.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz.

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Des weiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge von mindestens einer der erfindungsgemäßen Verbindungen.

Weiterer Gegenstand der vorliegenden Erfindung ist ein Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin All-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- aq.: wässrige Lösung
- ber.: Berechnet
- DCI: direkte chemische Ionisation (bei MS)
- DMF: Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- Et: Ethyl
- gef.: Gefunden
- h: Stunde(n)
- HPLC: Hochdruck-, Hochleistungsflüssigchromatographie
- HRMS: hochaufgelöste Massenspektrometrie
- konz.: konzentriert
- LC/MS: Flüssigchromatographie-gekoppelte Massenspektrometrie
- LiHMDS: Lithiumhexamethyldisilazid
- Me: Methyl
- min: Minute(n)
- MS: Massenspektrometrie
- NMR: Kernresonanzspektrometrie
- Pd₂dba₃: Tris-(dibenzylidenaceton)-dipalladium
- Ph: Phenyl
- RT: Raumtemperatur
- Rₜ: Retentionszeit (bei HPLC)
- t-Bu: tert.-Butyl
- THF: Tetrahydrofuran
- UV: Ultraviolett-Spektrometrie
- v/v: Volumen zu Volumen-Verhältnis (einer Lösung)
- XPHOS: Dicyclohexyl-(2',4',6'-triisopropylbiphenyl-2-yl)-phosphin

### LC/MS-Methoden:

### Methode 1 (LC-MS):

Instrument: Waters ACQUITY SQD UPLC System; Säule: Waters Acquity UPLC HSS T3 1,8µ 50 x 1mm; Eluent A: 11 Wasser + 0.25 ml 99%ige Ameisensäure , Eluent B: 11 Acetonitril + 0.25 ml 99%ige Ameisensäure; Gradient: 0.0 min 90% A → 1.2 min 5% A → 2.0 min 5% A; Ofen: 50°C; Fluss: 0.40 ml/min; UV-Detektion: 210 - 400 nm.

### Methode 2 (LC-MS):

Gerätetyp MS: Waters ZQ; Gerätetyp HPLC: Agilent 1100 Series; UV DAD; Säule: Thermo Hypersil GOLD 3 µ 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 100% A → 3.0 min 10% A → 4.0 min 10% A, Ofen: 55°C; Fluss 2ml/min; UV-Detektion: 210 nm.

### Methode 3 (LC-MS):

Instrument: Waters ACQUITY SQD UPLC System; Säule: Waters Acquity UPLC HSS T3 1.8 µ 30 x 2 mm; Eluent A: 11 Wasser + 0.25 ml 99%ige Ameisensäure , Eluent B: 11 Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Ausgangsverbindungen und Intermediate:

### Beispiel 1A

### 1-(2-Fluorbenzyl)-1H-pyrazolo[3,4-b]pyridin-3-carboximidamid-Hydrochlorid

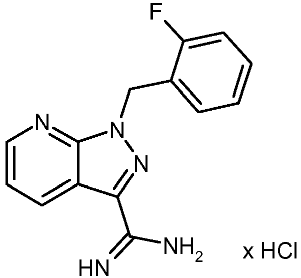

Die Synthese dieser Verbindung ist beschrieben in WO 2003/095451, Beispiel 6A.

### Beispiel 2A

### 2,6-Dichlor-5-fluornicotinamid

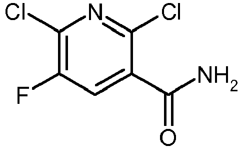

Eine Suspension aus 25 g (130.90 mmol) 2,6-Dichlor-5-fluor-3-cyanopyridin in konz. Schwefelsäure (125 ml) wurde 1 h bei 60-65°C gerührt. Nach Abkühlen auf RT wurde der Kolbeninhalt auf Eiswasser gegossen und dreimal mit Essigsäureethylester (je 100 ml) extrahiert. Die vereinigten organischen Phasen wurden mit Wasser (100 ml) und anschliessend mit gesättigter wässriger Natriumhydrogencarbonat-Lösung (100 ml) gewaschen, getrocknet und am Rotationsverdampfer eingeengt. Das erhaltene Material wurde am Hochvakuum getrocknet.

Ausbeute: 24.5 g (90 % d. Theorie)

¹H-NMR (400 MHz, DMSO-d₆): δ = 7.95 (br s, 1H), 8.11 (br s, 1H), 8.24 (d, 1H).

### Beispiel 3A

### 2-Chlor-5-fluornicotinamid

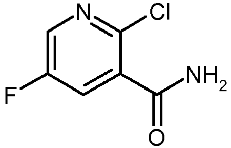

Zu einer Suspension von 21.9 g (335.35 mmol) Zink in Methanol (207 ml) wurden bei RT 44 g (210.58 mmol) 2,6-Dichlor-5-fluornicotinamid gegeben. Danach wurde mit Essigsäure (18.5 ml) versetzt und unter Rühren für 24 h zum Rückfluss erhitzt. Danach wurde der Kolbeninhalt vom Zink dekantiert und Essigsäureethylester (414 ml) sowie gesättigte wässrige Natriumhydrogencarbonat-Lösung (414 ml) zugegeben und intensiv ausgerührt. Anschliessend wurde über Kieselgur abgesaugt und dreimal mit Essigsäureethylester (je 517 ml) nachgewaschen. Die organische Phase wurde abgetrennt und die wässrige Phase mit Essigsäureethylester (258 ml) gewaschen. Die vereinigten organischen Phasen wurden einmal mit gesättigter wässriger Natriumhydrogencarbonat-Lösung (414 ml) gewaschen, getrocknet und im Vakuum eingeengt. Die so erhaltenen Kristalle wurden mit Dichlormethan (388 ml) versetzt und 20 min. ausgerührt. Es wurde erneut abgesaugt und mit Diethylether nachgewaschen und trockengesaugt.

Ausbeute: 20.2 g (53 % d. Theorie)

¹H-NMR (400 MHz, DMSO-d₆): δ = 7.87 (br s, 1H), 7.99 (dd, 1H), 8.10 (br s, 1H), 8.52 (d, 1H).

### Beispiel 4A

### 2-Chlor-5-fluornicotinonitril

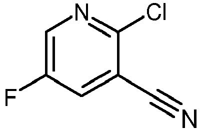

Eine Suspension von 46.2 g (264.66 mmol) 2-Chlor-5-fluornicotinamid in Dichlormethan (783 ml) wurde mit 81.2 ml (582.25 mmol) Triethylamin versetzt und auf 0°C gekühlt. Unter Rühren wurden dann 41.12 ml (291.13 mmol) Trifluoressigsäureanhydrid langsam zugetropft und 1.5 h bei 0°C nachgerührt. Die Reaktionslösung wurde danach zweimal mit gesättigter wässriger Natriumhydrogencarbonat-Lösung (je 391 ml) gewaschen, getrocknet und im Vakuum eingeengt.

Ausbeute: 42.1 g (90 % d. Theorie).

¹H-NMR (400 MHz, DMSO-d₆): δ = 8.66 (dd, 1H), 8.82 (d, 1H).

### Beispiel 5A

### 5-Fluor-1H-pyrazolo[3,4-b]pyridin-3-amin

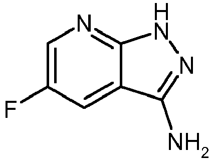

Eine Suspension aus 38.5 g (245.93 mmol) 2-Chlor-5-fluornicotinonitril wurde in 1,2-Ethandiol (380 ml) vorgelegt und danach mit Hydrazinhydrat (119.6 ml, 2.459 mol) versetzt. Es wurde unter Rühren für 4 h zum Rückfluss erhitzt. Beim Abkühlen fiel das Produkt aus. Die gelben Kristalle wurden mit Wasser (380 ml) versetzt und 10 min. bei RT ausgerührt. Anschliessend wurde die Suspension über eine Fritte abgesaugt, mit Wasser (200 ml) und mit -10°C-kaltem THF (200 ml) nachgewaschen. Der Rückstand wurde im Hochvakuum über Phosphorpentoxid getrocknet.

Ausbeute: 22.8 g (61 % d. Theorie)

¹H-NMR (400 MHz, DMSO-d₆): δ = 5.54 (s, 2H), 7.96 (dd, 1H), 8.38 (m, 1H), 12.07(m, 1H).

### Beispiel 6A

### 5-Fluor-3-iod-1H-pyrazolo[3,4-b]pyridin

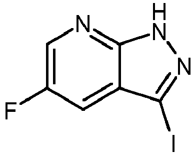

In THF (329 ml) wurden 10 g (65.75 mmol) 5-Fluor-1H-pyrazolo[3,4-b]pyridin-3-amin vorgelegt und auf 0°C abgekühlt. Anschliessend wurden 16.65 ml (131.46 mmol) Bortrifluorid-Diethylether-Komplex langsam zugesetzt. Die Reaktionsmischung wurde weiter auf -10°C abgekühlt. Danach wurde eine Lösung von 10.01 g (85.45 mmol) Isopentylnitrit in THF (24.39 ml) langsam zugefügt und weitere 30 min nachgerührt. Die Mischung wurde mit kaltem Diethylether (329 ml) verdünnt und der entstandene Feststoff abfiltriert. Das so hergestellte Diazoniumsalz wurde portionsweise in eine 0°C kalte Lösung von 12.81 g (85.45 mmol) Natriumiodid in Aceton (329 ml) gegeben und die Mischung 30 min bei RT nachgerührt. Die Reaktionsmischung wurde auf Eiswasser (1.81) gegeben und zweimal mit Essigsäureethylester (je 487 ml) extrahiert. Die gesammelten organischen Phasen wurden mit gesättigter wässriger Natriumchorid-Lösung (244 ml) gewaschen, getrocknet, filtriert und eingeengt. Man erhielt 12.1 g (86%-ige Reinheit, 60 % d. Th.) der gewünschten Verbindung als Feststoff. Das Rohprodukt wurde ohne weitere Reinigung umgesetzt.

LC-MS (Methode 1): Rₜ = 1.68 min; MS (ESIpos): m/z = 264 (M+H)⁺

### Beispiel 7A

### 5-Fluor-1-(2-fluorbenzyl)-3-iod-1H-pyrazolo[3,4-b]pyridin

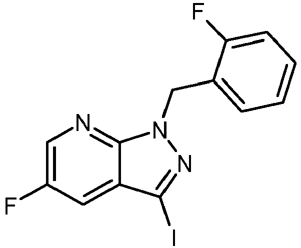

In DMF (217 ml) wurden 12.1 g (39.65 mmol) der Verbindung aus Beispiel 6A vorgelegt und anschließend 8.25 g (43.62 mmol) 2-Fluorbenzylbromid sowie 14.21 g (43.62 mmol) Cäsiumcarbonat zugefügt. Die Mischung wurde zwei Stunden bei RT gerührt. Anschließend wurde die Reaktionsmischung auf Wasser (1.171) gegeben und zweimal mit Essigsäureethylester (502 ml) extrahiert. Die gesammelten organischen Phasen wurden mit gesättigter wässriger Natriumchlorid-Lösung (335 ml) gewaschen, getrocknet, filtriert und eingeengt. Der Rückstand wurde an Kieselgel (Laufmittel: Petrolether/Essigsäureethylester 97:3) chromatographiert und die Produktfraktionen eingeengt. Man erhielt 9.0 g (61 % d. Th.) der gewünschten Verbindung als Feststoff. Der Feststoff wurde in Essigsäureethylester aufgenommen und mit 10%-iger wässriger Natriumthiosulfatlösung und danach mit gesättigter wässriger Natriumchlorid-Lösung gewaschen, getrocknet und eingeengt.

LC-MS (Methode 2): Rₜ = 2.57 min

MS (ESIpos): m/z = 372 (M+H)⁺

¹H-NMR (400 MHz, DMSO-d₆): δ = 5.73 (s, 2H), 7.13 - 7.26 (m, 3H), 7.33 - 7.41 (m, 1H), 7.94 (dd, 1H), 8.69 - 8.73 (m, 1H).

### Beispiel 8A

### 5-Fluor-1-(2-fluorbenzyl)-1H-pyrazolo[3,4-b]pyridin-3-carbonitril

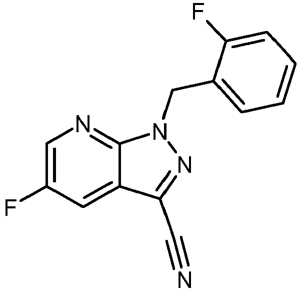

Eine Suspension aus 16.03 g (43.19 mmol) 5-Fluor-1-(2-fluorbenzyl)-3-iod-1H-pyrazolo[3,4-b]pyridin (Beispiel 7A) und 4.25 g (47.51 mmol) Kupfercyanid wurden in DMSO (120 ml) vorgelegt und 2 h bei 150°C gerührt. Nach Abkühlen wurde der Kolbeninhalt auf ca. 40°C abgekühlt, auf eine Lösung aus konz. Ammoniakwasser (90 ml) und Wasser (500 ml) gegossen, mit Essigsäureethylester (200 ml) versetzt und kurz ausgerührt. Die wässrige Phase wurde abgetrennt und noch zweimal mit Essigsäureethylester (je 200 ml) extrahiert. Die vereinigten organischen Phasen wurden zweimal mit 10%-iger wässriger Natriumchlorid-Lösung (je 100 ml) gewaschen, über Natriumsulfat getrocknet und im Vakuum eingeengt. Das Rohprodukt wurde ohne weitere Reinigung umgesetzt.

Ausbeute: 11.1 g (91 % d. Theorie)

¹H-NMR (400 MHz, DMSO-d₆): δ = 5.87 (s, 2H), 7.17 - 7.42 (m, 4H), 8.52 (dd, 1H), 8.87 (dd, 1H).

### Beispiel 9A

### 5-Fluor-1-(2-fluorbenzyl)-1H-pyrazolo[3,4-b]pyridin-3-carboximidamid-Acetat

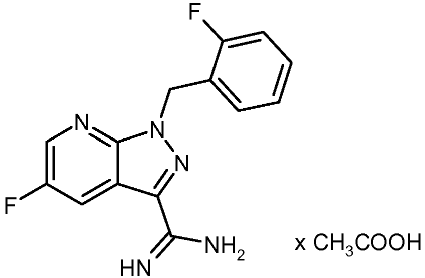

Zu 2.22 g (41.07 mmol) Natriummethanolat in Methanol (270 ml) wurden 11.1 g (41.07 mmol) 5-Fluor-1-(2-fluorbenzyl)-1H-pyrazolo[3,4-b]pyridin-3-carbonitril (Beispiel 8A) gegeben und 2 h bei RT gerührt. Danach wurden 2.64 g (49.29 mmol) Ammoniumchlorid und Essigsäure (9.17 ml) zugegeben und über Nacht zum Rückfluss erhitzt. Danach wurde bis zur Trockene eingeengt und der Rückstand in Wasser (100 ml) und Essigsäureethylester (100 ml) aufgenommen und mit 2N Natronlauge auf pH 10 gestellt. Es wurde für ca. 1 h bei RT intensiv gerührt. Die erhaltene Suspension wurde abgesaugt und mit Essigsäureethylester (100 ml), Wasser (100 ml) und nochmals Essigsäureethylester (100 ml) nachgewaschen. Der Rückstand wurde über Phosphorpentoxid im Hochvakuum getrocknet.

Ausbeute: 9.6 g (78 % d. Th.)

MS (ESIpos): m/z = 288 (M+H)⁺

¹H-NMR (400 MHz, DMSO-d₆): δ = 1.85 (s, 3H), 5.80 (s, 2H), 7.14 - 7.25 (m, 3H), 7.36 (m, 1H), 8.42 (dd, 1H), 8.72 (dd, 1H).

### Beispiel 10A

### Methyl-3,3-dicyan-2,2-dimethylpropanoat

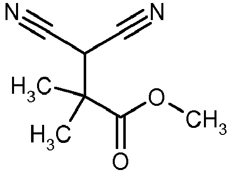

In THF (91 ml) wurden 1.816 g (45.411 mmol) Natriumhydrid (60% in Mineralöl) langsam mit 3 g (45.411 mmol) Malonsäuredinitril versetzt. Anschliessend wurden 5.876 ml (45.411 mmol) Methyl-2-brom-2-methylpropanoat zugegeben und es wurde über Nacht bei Raumtemperatur gerührt. Danach wurden nochmals 5.876 ml (45.411 mmol) Methyl-2-brom-2-methylpropanoat zugegeben und es wurde über Nacht auf 50°C erhitzt. Dann wurden abermals 1.762 ml (13.623) mmol) Methyl-2-brom-2-methylpropanoat zugegeben und es wurde weitere 4h auf 50°C erhitzt. Der Ansatz wurde dann mit gesättigter wässriger Natriumhydrogencarbonat-Lösung versetzt und dreimal mit Essigsäureethylester extrahiert. Die vereinigten organischen Phasen wurden mit gesättigter wässriger Natriumchlorid-Lösung gewaschen, über Natriumsulfat getrocknet, filtriert und bis zur Trockene eingeengt. Man erhielt 8.9 g Rohprodukt, welches per Chromatographie an Kieselgel (Cyclohexan-Essigsäureethylester 4:1) gereinigt wurde.

Ausbeute: 6.47 g (85% d. Th.)

¹H-NMR (400 MHz, DMSO-d₆): δ [ppm] = 1.40 (s, 6H), 3.74 (s, 3H), 5.27 (s, 1H).

### Beispiel 11A

### Methyl-3-bromtetrahydrofuran-3-carboxylat

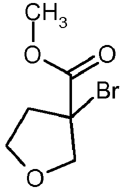

Es wurden 5.0 g (38.419 mmol) Methyltetrahydrofuran-3-carboxylat (Herstellung erfolgte analog zu: J. Org. Chem. 1996, 2690) in 200 ml THF gelöst und auf -78°C abgekühlt und anschliessend mit 76.83 ml einer 1M Lösung von Bis-(trimethylsilyl)-lithiumamid in THF versetzt. Nach 30 min bei -78°C wurden 10.26 g (57.63 mmol) N-Bromsuccinimid in 50 ml THF suspendiert langsam zugegeben. Danach wurde ließ man über Nacht auf RT erwärmen. Der Ansatz wurde mit Wasser versetzt und mit Essigsäureethylester extrahiert. Die Phasen wurden getrennt und die wässrige Phase wurde noch zweimal mit Essigsäureethylester extrahiert. Die vereinigten organischen Phasen wurden mit gesättigter wässriger Natriumchlorid-Lösung gewaschen und anschliessend über Natriumsulfat getrocknet, filtriert und eingeengt. Das Rohprodukt wurde mittels Chromatographie an Kieselgel (Laufmittel: Dichlormethan) gereinigt. Man erhielt 491 mg (6 % d. Th.) der Zielverbindung.

¹H-NMR (400 MHz, CDCl₃): δ [ppm] = 2.49 (ddd, 1H), 2.74 (ddd, 1H), 3.83 (s, 3H), 4.03-4.10 (m, 1H), 4.11-4.17 (m, 2H), 4.31 (d, 1H).

### Beispiel 12A

### Methyl-3-(dicyanmethyl)tetrahydrofuran-3-carboxylat

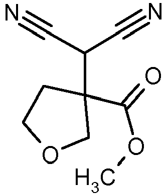

440 mg (11.00 mmol) Natriumhydrid (60% in Mineralöl) wurden in 30 ml THF vorgelegt und portionsweise mit 726 mg (11.00 mmol) Malonsäuredinitril versetzt. Danach wurden 2.3 g (11.00 mmol) der unter Beispiel 11A erhaltenen Verbindung in THF (50 ml) zugegeben. Es wurde 6 h bei RT gerührt und anschliessend über Nacht auf 50°C erhitzt. Nach Abkühlen wurde der Ansatz mit gesättigter wässriger Natriumhydrogencarbonat-Lösung versetzt und dreimal mit Essigsäureethylester extrahiert. Die vereinigten organischen Phasen wurden mit gesättigter wässriger Natriumchlorid-Lösung gewaschen und anschliessend über Natriumsulfat getrocknet, filtriert und eingeengt. Der Rückstand (2.66 g) wurde 1 h am Hochvakuum getrocknet und anschliessend ohne weitere Reinigung umgesetzt.

### Beispiel 13A

### 2-Methoxy-4-methyl-6-oxo-1, 4, 5, 6-tetrahydropyridin-3-carbonitril

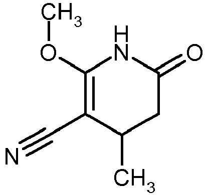

Die Synthese der Verbindung ist beschrieben: Heterocycles, 1985; 1135 - 1141.

### Beispiel 14A

### 2-Methoxy-6-oxo-4-(trifluormethyl)-1,4,5,6-tetrahydropyridin-3-carbonitril

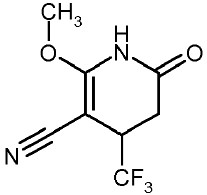

7.47 g (138.39 mmol) Natriummethanolat in Methanol (85 ml) wurden unter Eiskühlung vorgelegt und portionsweise mit 6.04 g (91.44 mmol) Malonodinitril versetzt. Anschliessend wurden unter Rühren 11.84 g (76.84 mmol) Methyl-4,4,4-trifluorocrotonat zugetropft, 30 min bei Raumtemperatur gerührt und danach 1 h zum Rückfluss erhitzt. Im Anschluss wurde im Vakuum bis zur Trockene eingeengt. Der Rückstand wurde mit Wasser versetzt und viermal mit Essigsäureethylester extrahiert. Die vereinigten organischen Phasen wurden über Natriumsulfat getrocknet, filtriert und eingeengt. Weitere Reinigung erfolgte durch Chromatographie an Kieselgel (Cyclohexan-Essigsäureethylester 3:1). Man erhielt 1.95 g der Zielverbindung (11 % d. Th.).

LC-MS (Methode 1): Rₜ = 0.61 min; MS (ESIpos): m/z = 221 (M+H)⁺

### Beispiel 15A

### 5-Fluor-3-iod-1H-pyrazolo[3,4-b]pyridin

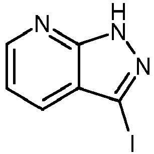

Die Synthese ist beschrieben in in WO 2006/130673, Schema D.

### Beispiel 16A

### 3-Iod-1-(3,3,4,4,4-pentafluorbutyl)-1H-pyrazolo[3,4-b]pyridin

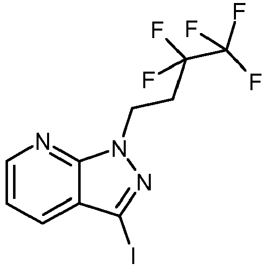

In DMF (170 ml) wurden 10.00 g (40.813 mmol) Beispiel 15A vorgelegt und anschließend 12.30 g (44.894 mmol) 1,1,1,2,2-Pentafluor-4-iodbutan in DMF (30 ml) sowie 14.628 g (44.894 mmol) Cäsiumcarbonat zugefügt. Die Mischung wurde für 2 Tage bei RT gerührt. Anschließend wurden erneut 12.30 g (44.894 mmol) 1,1,1,2,2-Pentafluor-4-iodbutan sowie 14.628 g (44.894 mmol) Cäsiumcarbonat zugegeben und für 2 Tage bei RT gerührt. Danach wurden 3.485 g (12.720 mmol) 1,1,1,2,2-Pentafluor-4-iodbutan und 4.145 g (12.720 mmol) Cäsiumcarbonat zugegeben und über Nacht bei RT gerührt. Nach dieser Zeit wurden wiederum 5.00 g (18.250 mmol) 1,1,1,2,2-Pentafluor-4-iodbutan und 5.946 g (18.250 mmol) Cäsiumcarbonat zugegeben und es wurde 6 Tage bei Raumtemperatur gerührt. Anschließend wurde für 2 Tage bei 70°C gerührt.. Es wurde von Feststoffen abgesaugt, mit DMF nachgewaschen und anschliessend im Hochvakuum eingeengt. Der Rückstand wurde mittels präparativer HPLC gereinigt (Methanol:Wasser (mit 0.1% Ameisensäure) - Gradient). Man erhielt 5.48 g (34 % d. Th.) der Titelverbindung als Feststoff.

LC-MS (Methode 3): Rₜ = 1.23 min

MS (ESIpos): m/z = 392 (M+H)⁺

1H-NMR (400 MHz, DMSO-d6): δ = 2.87-3.00 (m, 2H), 4.81 (t, 2H), 7.33 (dd, 1H), 7.97 (dd, 1H), 8.65 (dd, 1H).

### Beispiel 17A

### 1-(3,3,4,4,4-Pentafluorbutyl)-1H-pyrazolo[3,4-b]pyridin-3-carbonitril

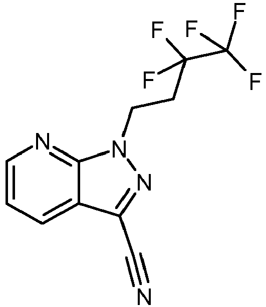

Eine Suspension aus 5.480 g (14.012 mmol) Beispiel 16A und 1.380 g (15.414 mmol) Kupfer-(I)-cyanid wurden in DMSO (50 ml) vorgelegt und 3 h bei 150°C gerührt. Nach Abkühlen wurde über Celite filtriert und mit Ethylacetat und THF nachgewaschen. Danach wurde viermal mit einer Lösung aus ges. wässr. Ammoniumchloridlösung und konz. Ammoniakwasser (3:1, v/v) und anschliessend mit ges. wässr. Natriumchloridlösung gewaschen. Die organische Phase wurde über Natriumsulfat getrocknet, filtriert und eingeengt und anschliessend am Hochvakuum getrocknet.

Ausbeute: 3.59 g (88 % d. Theorie)

LC-MS (Methode 1): Rₜ = 1.04 min

MS (ESIpos): m/z = 291 (M+H)⁺

1H-NMR (400 MHz, DMSO-d6): δ = 2.97-3.10 (m, 2H), 4.94 (t, 2H), 7.55 (dd, 1H), 8.51 (dd, 1H), 8.81 (dd, 1H).

### Beispiel 18A

### 1-(3,3,4,4,4-Pentafluorbutyl)-1H-pyrazolo[3,4-b]pyridin-3-carboximidamid-Acetat

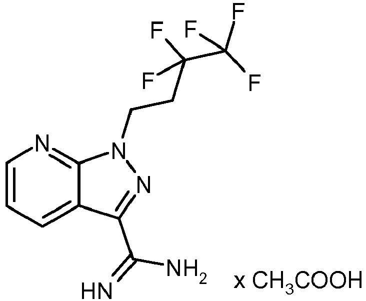

Zu 0.668 g (12.371 mmol) Natriummethanolat in Methanol (40 ml) wurden 3.59 g (12.371 mmol) Beispiel 17A in Methanol (20 ml) gegeben und 2 h bei RT gerührt. Danach wurden 0.794 g (14.845 mmol) Ammoniumchlorid und Essigsäure (2.762 ml) zugegeben und über Nacht zum Rückfluss erhitzt. Danach wurde bis zur Trockene eingeengt und der Rückstand mit Essigsäureethylester und 1N Natronlauge versetzt. Es wurde für ca. 1 h bei RT intensiv gerührt. Der erhaltene Feststoff wurde abgesaugt und mit Essigsäureethylester und Wasser nachgewaschen. Der Rückstand im Hochvakuum getrocknet. Man erhielt 0.507 g (11 % d. Theorie, Reinheit 100%). Bei den Waschfraktionen wurden die Phasen getrennt und die wässrige Phase wurde zweimal mit Ethylacetat extrahiert. Die vereinigten organischen Phasen wurden mit Wasser und ges. wässr. Natriumchloridlösung gewaschen, über Natriumsulfat getrocknet, filtriert und eingeengt und anschliessend am Hochvakuum getrocknet. Man erhielt so weitere 2.76 g (43 % d. Theorie, Reinheit 71 %).

LC-MS (Methode 1): Rₜ = 0.58 min

MS (ESIpos): m/z = 308 (M+H)⁺

1H-NMR (400 MHz, DMSO-d₆): δ = 1.84 (s, 3H), 2.95-3.08 (m, 2H), 4.85 (t, 2H), 7.39 (dd, 1H), 8.63-8.67 (m, 2H).

### Beispiel 19A

### 5-Fluor-3-iod-1-(3,3,4,4,4-pentafluorbutyl)-1H-pyrazolo[3,4-b]pyridin

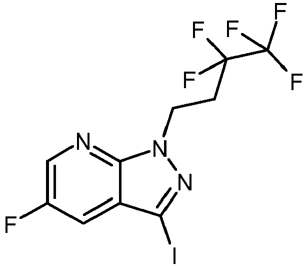

In DMF (100 ml) wurden 5.0 g (19.010 mmol) 5-Fluor-3-iod-1H-pyrazolo[3,4-b]pyridinvorgelegt und anschließend 20.83 g (76.042 mmol) 1,1,1,2,2-Pentafluor-4-iodbutan sowie 14.86 g (45.65 mmol) Cäsiumcarbonat und 0.63 g (3.802 mmol) Kaliumiodid zugefügt. Die Mischung wurde über Nacht bei 140°C gerührt. Anschließend wurde abgekühlt und mit einem Vorversuch vereinigt, der analog von 200 mg 5-Fluor-3-iod-1H-pyrazolo[3,4-b]pyridin ausging. Es wurde von Feststoffen abgesaugt, mit DMF nachgewaschen und anschliessend im Hochvakuum eingeengt. Der Rückstand wurde mittels präparativer HPLC gereinigt (Methanol:Wasser - Gradient). Man erhielt 4.34 g (52 % d. Th.) der Titelverbindung als Feststoff.

LC-MS (Methode 3): Rₜ = 1.30 min

MS (ESIpos): m/z = 410 (M+H)⁺

1H-NMR (400 MHz, DMSO-d6): δ = 2.84-3.00 (m, 2H), 4.79 (t, 2H), 7.93 (dd, 1H), 8.71 (dd, 1H).

### Beispiel 20A

### 5-Fluor-1-(3,3,4,4,4-pentafluorbutyl)-1H-pyrazolo[3,4-b]pyridin-3-carbonitril

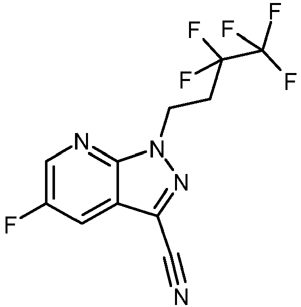

Eine Suspension aus 4.34 g (10.609 mmol) Beispiel 19A und 1.045 g (11.670 mmol) Kupfer-(I)-cyanid wurden in DMSO (30 ml) vorgelegt und 2 h bei 150°C gerührt. Nach Abkühlen wurde der Ansatz über Celite filtriert, mit Essigsäureethylester und THF nachgewaschen und anschliessend viermal mit einer Lösung aus ges. wässriger Ammoniumchloridlösung und konz. Ammoniakwasser (3:1 v/v) extrahiert. Die vereinigten organischen Phasen wurden mit ges. wässriger Natriumchlorid-Lösung gewaschen, über Natriumsulfat getrocknet, filtriert und im Vakuum eingeengt.

Ausbeute: 3.19 g (97 % d. Theorie)

1H-NMR (400 MHz, DMSO-d6): δ = 2.94-3.09 (m, 2H), 4.93 (t, 2H), 8.54 (dd, 1H), 8.88 (dd, 1H).

### Beispiel 21A

### 5-Fluor-1-(3,3,4,4,4-pentafluorbutyl)-1H-pyrazolo[3,4-b]pyridin-3-carboximidamid-Acetat

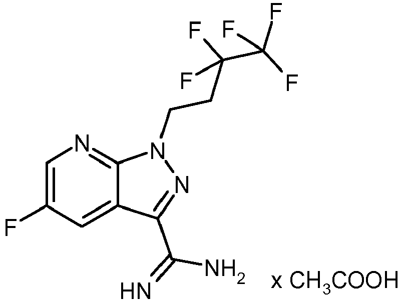

Zu 0.559 g (10.351 mmol) Natriummethanolat in Methanol (25 ml) wurden 3.19 g (10.351 mmol) Beispiel 20A gegeben und 2 h bei RT gerührt. Danach wurden 0.664 g (12.421 mmol) Ammoniumchlorid und Essigsäure (2.31 ml) zugegeben und über Nacht zum Rückfluss erhitzt. Danach wurde bis zur Trockene eingeengt und der Rückstand mit Essigsäureethylester und mit 1N Natronlauge versetzt. Die Phasen wurden getrennt. Die wässrige Phase wurde nochmals mit Essigsäureethylester extrahiert. Die vereinigten organischen Phasen wurden vereinigt und eingeengt.

Ausbeute: 2.67 g (37 % d. Theorie, Reinheit ca. 56 %)

LC-MS (Methode 1): Rₜ = 0.68 min

MS (ESIpos): m/z = 326 (M+H)⁺

### Ausführungsbeisniele:

### Beispiel 1

### 4-Amino-2-[1-(2-fluorbenzyl)-1H-pyrazolo[3,4-b]pyridin-3-yl]-5,7-dihydro-6H-pyrrolo[2,3-d]pyrimidin-6-on

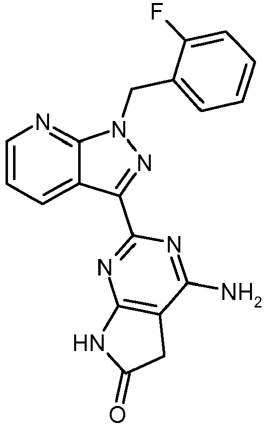

### Stufe a)

1.51 g (37.84 mmol) Natriumhydrid (60 %ig in Mineralöl) wurden in 10 ml DMSO vorgelegt. Danach wurde unter Kühlung 2.5 g (37.843 mmol) Malonsäuredinitril in DMSO (10 ml) langsam zugetropft und 10 min gerührt. Anschliessend wurde bei Raumtemperatur 3.582 ml (37.843 mmol) Bromessigsäuremethylester in DMSO (10 ml) zugetropft. Es wurde weitere 2h bei Raumtemperatur gerührt. Dann wurde durch Zugabe von gesättigter wässriger Ammoniumchlorid-Lösung abgebrochen und mit Essigsäureethylester versetzt. Die Phasen wurden getrennt und die wässrige Phase wurde noch einmal mit Essigsäureethylester extrahiert. Die vereinigten organischen Phasen wurden noch einmal mit gesättigter wässriger Ammoniumchlorid-Lösung gewaschen. Anschliessend wurde über Natriumsulfat getrocknet, filtriert und bis zur Trockene eingeengt. Das Rohprodukt wurde ohne weitere Reinigung im Schritt b) eingesetzt:

### Stufe b)

1.04 g (3.403 mmol) Beispiel 1A wurden in tert.-Butanol vorgelegt und mit 458 mg (4.083 mmol) Kalium-tert.-butylat versetzt. Anschliessend wurden 470 mg (3.403 mmol) des Rohproduktes aus Sutfe a) in tert.-Butanol zugegeben und die Mischung wurde über Nacht zum Rückfluss erhitzt. Nach Abkühlen wurde mit Wasser und Essigsäureethylester versetzt und die Phasen getrennt. Die wässrige Phase wurde zweimal mit Essigsäureethylester extrahiert. Die vereinigten organischen

Phasen wurden noch einmal mit Wasser und einmal mit gesättigter wässriger Natriumchlorid-Lösung gewaschen. Anschliessend wurde über Natriumsulfat getrocknet, filtriert und bis zur Trockene eingeengt. Der Rückstand wurde mittels präparativer HPLC gereinigt (Acetonitril:Wasser (+0.05 % Ameisensäure) - Gradient). Nach Einengen der Produktfraktionen wurde mit DMF, Wasser und Acetonitril versetzt, wobei sich ein unlöslicher Rückstand bildete, der abfiltriert wurde. Nach Waschen des Feststoffs mit Acetonitril wurde 23 mg der Zielverbindung erhalten (2 % d. Th.).

LC-MS (Methode 1): Rₜ = 0.82 min; MS (ESIpos): m/z = 376 (M+H)⁺

¹H-NMR (400 MHz, DMSO-d₆): δ [ppm] = 3.34 (s, 2H), 5.81 (s, 2H), 6.85 (s br, 2H), 7.13-7.25 (m, 3H), 7.33-7.40 (m, 2H), 8.63 (dd, 1H), 8.99 (dd, 1H), 10.95 (s br, 1H).

### Beispiel 2

### 4-Amino-2-[1-(2-fluorbenzyl)-1H-pyrazolo[3,4-b]pyridin-3-yl]-5,5-dimethyl-5,7-dihydro-6H-pyrrolo[2,3-d]pyrimidin-6-on

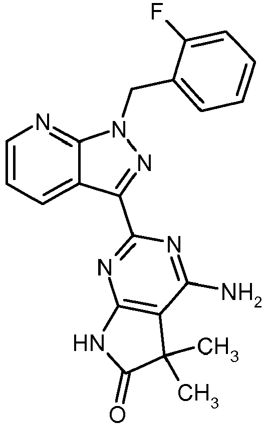

5.887 g (19.256 mmol) Beispiel 1A wurden in tert.-Butanol (50 ml) vorgelegt und mit 2.593 g (23.107 mmol) Kalium-tert.-butylat versetzt. Anschliessend wurden 3.2 g (19.256 mmol) Beispiel 10A in tert.-Butanol (25 ml) zugetropft und die Mischung über Nacht zum Rückfluss erhitzt. Am nächsten Tag wurden nochmal 0.64 g (3.851 mmol) Beispiel 10A zugegeben und es wurde für einen weiteren Tag zum Rückfluss erhitzt. Nach Abkühlen wurde ein Niederschlag abfiltriert, welcher mit Diethylether nachgewaschen wurde. Anschliessend wurde in Wasser aufgeschlämmt und ein weiteres Mal abfiltriert und mit Diethylether nachgewaschen. Nach Trocknung im Hochvakuum konnten 6.65 g der Zielverbindung erhalten (85 % d. Th.) werden.

LC-MS (Methode 1): Rₜ = 0.90 min; MS (ESIpos): m/z = 404 (M+H)⁺

¹H-NMR (400 MHz, DMSO-d₆): δ [ppm] = 1.35 (s, 6H), 5.82 (s, 2H), 6.82 (br s, 2H), 7.14-7.25 (m, 3H), 7.33-7.40 (m, 2H), 8.63 (dd, 1H), 9.03 (dd, 1H), 10.98 (s br, 1H).

### Beispiel 3

### 4-Amino-2-[5-fluor-1-(2-fluorbenzyl)-1H-pyrazolo[3,4-b]pyridin-3-yl]-5,5-dimethyl-5,7-dihydro-6H-pyrrolo[2,3-d]pyrimidin-6-on

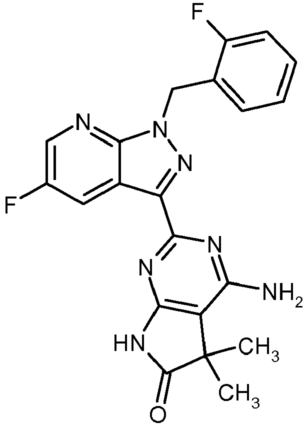

In Analogie zur Darstellung von Beispiel 2 wurden 4.18 g (12.035 mmol) Beispiel 9A mit 2.20 g (13.239 mmol) Beispiel 10A umgesetzt. Es wurden 3.72 g der Zielverbindung erhalten (73 % d. Th.).

LC-MS (Methode 1): Rₜ = 0.98 min; MS (ESIpos): m/z = 422 (M+H)⁺

¹H-NMR (400 MHz, DMSO-d₆): δ [ppm] = 1.34 (s, 6H), 5.81 (s, 2H), 6.85 (br s, 2H), 7.13-7.25 (m, 3H), 7.36 (m, 1H), 8.69 (dd, 1H), 8.84 (dd, 1H), 10.96 (s br, 1H).

### Beispiel 4

### 4'-Amino-2'-[1-(2-fluorbenzyl)-1H-pyrazolo[3,4-b]pyridin-3-yl]-4,5-dihydrospiro[furan-3,5'-pyrrolo[2,3-d]pyrimidin]-6'(7'H)-on

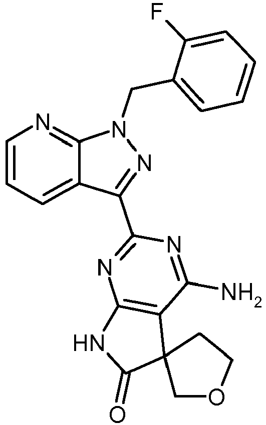

In Analogie zur Darstellung von Beispiel 2 wurden 2.257 g (7.382 mmol) Beispiel 1A mit 1.434 g (7.382 mmol) Beispiel 12A umgesetzt. Es wurden 566 mg der Zielverbindung erhalten (17 % d. Th.).

LC-MS (Methode 1): Rₜ = 0.84 min; MS (ESIpos): m/z = 432 (M+H)⁺

¹H-NMR (400 MHz, DMSO-d₆): δ [ppm] = 2.20-2.37 (m, 2H), 3.71 (d, 1H), 3.90 (q, 1H), 4.10 (d, 1H), 4.25-4.31 (m, 1H), 5.82 (s, 2H), 6.57 (br s, 2H), 7.12-7.25 (m, 3H), 7.33-7.41 (m, 2H), 8.64 (dd, 1H), 9.02 (dd, 1H), 11.96 (s br, 1H).

### Beispiel 5

### 4-Amino-2-[1-(2-fluorbenzyl)-1H-pyrazolo[3,4-b]pyridin-3-yl]-5-methyl-5,8-dihydropyrido[2,3-d]pyrimidin-7(6H)-on

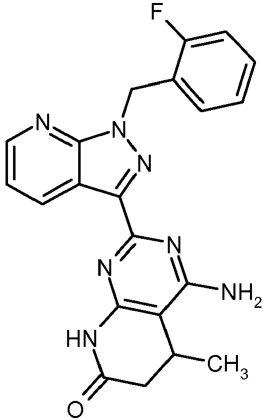

2.174 g (7.112 mmol) Beispiel 1A und 1.3 g (7.823 mmol) Beispiel 13A wurden in 20 ml Methanol vorgelegt und anschliessend bei Raumtemperatur portionsweise mit 422 mg (7.823 mmol) Natriummethanolat versetzt. Man rührte für 10 min bei Raumtemperatur und erhitzte anschliessend über Nacht zum Rückfluss. Nach Abkühlen wurde der Ansatz mit Essigsäure (0,5 ml) und Wasser (20 ml) versetzt und im Eisbad gekühlt. Der Niederschlag wurde abgesaugt, mit Wasser und Methanol gewaschen und anschliessend im Hochvakuum getrocknet. Es wurden 2.51 g der Zielverbindung erhalten (87 % d. Th.).

LC-MS (Methode 1): Rₜ = 0.85 min; MS (ESIpos): m/z = 404 (M+H)⁺

¹H-NMR (400 MHz, DMSO-d₆): δ [ppm] = 1.04 (d, 3H), 2.31 (d, 1H), 2.79 (dd, 1H), 3.13-3.19 (m, 1H), 5.81 (s, 2H), 6.93 (br s, 2H), 7.12-7.25 (m, 3H), 7.34-7.37 (m, 2H), 8.62 (dd, 1H), 9.14 (dd, 1H), 10.56 (s, 1H).

### Beispiel 6

### 4-Amino-2-[1-(2-fluorbenzyl)-1H-pyrazolo[3,4-b]pyridin-3-yl]-5-(trifluormethyl)-5,8-dihydropyrido[2,3-d]pyrimidin-7(6H)-on

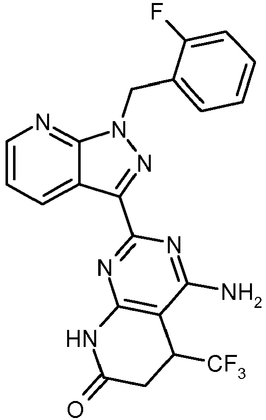

694 mg (2.271 mmol) Beispiel 1A und 500 mg (2.271 mmol) Beispiel 14A wurden in 10 ml t-Butanol vorgelegt und anschliessend bei Raumtemperatur portionsweise mit 305 mg (2.725 mmol) Kalium-tert.-butylat versetzt. Man rührte für 10 min bei Raumtemperatur und erhitzte anschliessend für 2 Tage zum Rückfluss. Nach Abkühlen wurde der Ansatz mit Wasser und Essigsäureethylester versetzt. Der Niederschlag wurde abgesaugt. Das Filtrat wurde eingeengt, mit wenig Essigsäureethylester und Diethylether versetzt und der entstandene Niederschlag abgesaugt. Die vereinigten Feststoffe aus beiden Teilschritten wurden anschliessend im Hochvakuum getrocknet. Es wurden 588 mg der Zielverbindung erhalten (53 % d. Th.).
LC-MS (Methode 1): Rt = 0.92 min; MS (ESIpos): m/z = 458 (M+H)⁺
1H-NMR (400 MHz, DMSO-d6): δ [ppm] = 2.63 (d, 1H), 3.19 (dd, 1H), 4.16-4.20 (m, 1H), 5.83 (s, 2H), 7.13-7.40 (m, 7H), 8.63 (dd, 1H), 9.15 (dd, 1H), 10.85 (s, 1H).

### Beispiel 7

### (Referenzbeispiel)

### 4-Amino-5,5-dimethyl-2-[1-(3,3,4,4,4-pentafluorbutyl)-1H-pyrazolo[3,4-b]pyridin-3-yl]-5,7-dihydro-6H-pyrrolo[2,3-d]pyrimidin-6-on

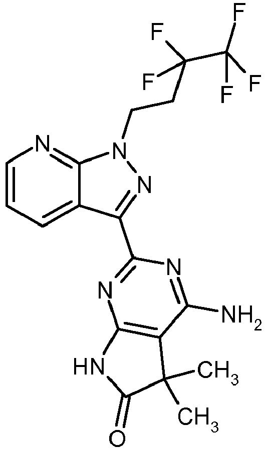

500 mg (1.361 mmol) Beispiel 18A wurden in tert.-Butanol (7.5 ml) vorgelegt und mit 183 mg (1.361 mmol) Kalium-tert.-butylat versetzt. Anschliessend wurden 226 mg (1.361 mmol) Beispiel 10A in tert.-Butanol (2.5 ml) zugetropft und die Mischung über Nacht zum Rückfluss erhitzt. Nach Abkühlen wurde mit Essigsäureethylester und Wasser versetzt, die Phasen getrennt und die wässrige Phase wurde zweimal mit Essigsäureethylester extrahiert. Die vereinigten organischen Phasen wurden mit ges. wässr. Natriumchloridlösung gewaschen, über Natriumsulfat getrocknet, filtriert und eingeengt. Der Rückstand wurde mit Methanol verrührt und von einem Feststoff abgesaugt. Dieser wurde kräftig mit Methanol nachgewaschen und die vereinigten Filtrate wurden eingeengt und anschliessend mittels präparativer HPLC gereinigt (Acetonitril:Wasser (+0.05 % Ameisensäure) - Gradient). Es wurden 127 mg der Titelverbindung erhalten (21 % d. Th.).
LC-MS (Methode 1): Rₜ = 0.93 min; MS (ESIpos): m/z = 442 (M+H)⁺
¹H-NMR (400 MHz, DMSO-d₆): δ [ppm] = 1.36 (s, 6H), 2.91-3.04 (m, 2H), 4.88 (t, 2H), 6.83 (br s, 2H), 7.38 (dd, 1H), 8.63 (dd, 1H), 9.02 (dd, 1H), 11.01 (s br, 1H).

### Beispiel 8

### (Referenzbeispiel)

### 4-Amino-2-[5-fluor-1-(3,3,4,4,4-pentafluorbutyl)-1H-pyrazolo[3,4-b]pyridin-3-yl]-5,5-dimethyl-5,7-dihydro-6H-pyrrolo[2,3-d]pyrimidin-6-on

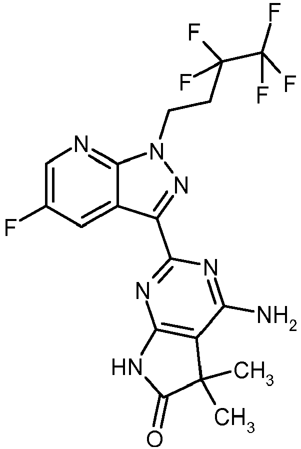

520 mg (1.350 mmol) Beispiel 21A wurden in tert.-Butanol (10 ml) vorgelegt und mit 181 mg (1.620 mmol) Kalium-tert.-butylat versetzt. Anschliessend wurden 224 mg (1.350 mmol) Beispiel 10A in tert.-Butanol (2.5 ml) zugegeben und die Mischung über Nacht zum Rückfluss erhitzt. Anschliessend wurden abermals 112 mg (0.675 mmol) Beispiel 10A zugegeben und es wurde für weitere 7.5 h zum Rückfluss erhitzt. Nach Abkühlen wurde mit Wasser und Ethanol versetzt und für 1 h im Ultraschallbad behandelt. Es entstand ein Niederschlag, welcher abgesaugt wurde und mit Wasser nachgewaschen wurde. Der Filterkuchen wurde mit wenig Ethanol (2-3 ml) durchrührt und abermals abgesaugt. Der Feststoff wurde am Hochvakuum getrocknet. Es wurden 212 mg der Titelverbindung erhalten (34 % d. Th.).
LC-MS (Methode 1): Rₜ = 1.01 min; MS (ESIpos): m/z = 460 (M+H)⁺
¹H-NMR (400 MHz, DMSO-d₆): δ [ppm] = 1.36 (s, 6H), 2.92-3.04 (m, 2H), 4.87 (t, 2H), 6.88 (br s, 2H), 8.71 (s br, 1H), 8.85 (dd, 1H), 11.01 (s br, 1H).

### B. Bewertung der pharmakologischen Wirksamkeit

Die pharmakologische Wirkung der erfindungsgemäßen Verbindungen kann in folgenden Assays gezeigt werden:

### B-1. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

Repräsentative IC₅₀-Werte für die erfindungsgemäßen Verbindungen sind in der nachstehenden Tabelle (Tabelle 1) wiedergegeben:

**Tabelle 1:**

| Beispiel Nr. | IC₅₀ [nM] |
|---|---|
| 2 | 48 |
| 3 | 9.3 |
| 4 | 24 |

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Werte (MEC = minimal effektive Konzentration) für die erfindungsgemäßen Verbindungen sind in der nachstehenden Tabelle (Tabelle 2) wiedergegeben:

**Tabelle 2:**

| Beispiel Nr. | MEC [µM] |
|---|---|
| 2 | 0.001 |
| 3 | 0.001 |
| 4 | 0.003 |

### B3. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
- Implantierbare Sender (Physiotel® Telemetrietransmitter)
- Empfänger (Physiotel® Receiver), die über einen Multiplexer (DSI Data Exchange Matrix ) mit einem
- Datenakquisitionscomputer
verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makroion - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p. ) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer lml/kg s.c.)

### Substanzen und Lösungen

Wenn nicht anders beschrieben werden die zu untersuchenden Substanzen jeweils einer Gruppe von Tieren (n = 6 ) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI ).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI ) online erfasst und entsprechend aufgearbeitet werden. Die Ablage der Daten erfolgt jeweils in einem hierfür eröffneten Ordner der die Versuchsnummer trägt.

Im Standardablauf werden über je 10 Sekunden Dauer gemessen:
- Systolischer Blutdruck (SBP)
- Diastolischer Blutdruck (DBP)
- Arterieller Mitteldruck (MAP)
- Herzfrequenz (HR)
- Aktivität (ACT).

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994

### C. Ausfuhrunssbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 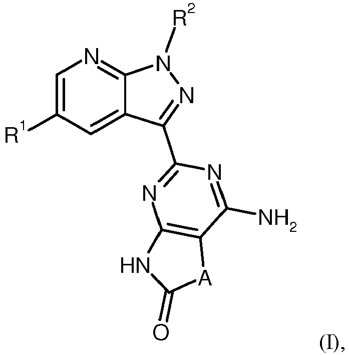 in welcher
A für (C₁-C₃)-Alkandiyl oder eine Gruppe der Formel 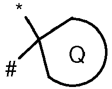 steht, wobei
* für die Anknüpfstelle an den Pyrimidinring steht,
# für die Anknüpfstelle an die Carbonylgruppe steht,
der Ring Q für einen 4- bis 6-gliedrigen Heterocyclus steht,
und
wobei (C₁-C₃)-Alkandiyl mit 1 oder 2 Substituenten unabhängig voneinander ausgewählt aus der Gruppe Fluor, Trifluormethyl, (C₁-Ca)-Alkyl, Hydroxy und Amino substituiert sein kann,
worin (C₁-C₄)-Alkyl mit 1 bis 3 Substituenten unabhängig voneinander ausgewählt aus der Gruppe Fluor, Trifluormethyl und Hydroxy substituiert sein kann,
R¹ für Wasserstoff oder Fluor steht,
R² für Benzyl steht,
wobei Benzyl mit 1 bis 3 Substituenten Fluor substituiert ist,
sowie ihre *N*-Oxide, Salze, Solvate, Salze der *N*-Oxide und Solvate der *N*-Oxide und Salze.

2. Verbindung der Formel (I) nach Anspruch 1, in welcher
A für Gruppe der Formel 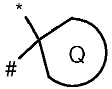 steht, wobei
* für die Anknüpfstelle an den Pyrimidinring steht,
# für die Anknüpfstelle an die Carbonylgruppe steht,
der Ring Q für einen Azetidinyl-, Oxetanyl-, Pyrrolidinyl-, Tetrahydrofuranyl-, Piperidinyl- oder Tetrahydropyranylring steht,
R¹ für Wasserstoff oder Fluor steht,
R² für Benzyl steht,
wobei Benzyl mit 1 bis 3 Substituenten Fluor substituiert ist,
sowie ihre Salze, Solvate und Solvate der Salze.

3. Verbindung der Formel (I) nach Anspruch 1, in welcher
A für (C₁-C₃)-Alkandiyl
wobei (C₁-C₃)-Alkandiyl mit 1 oder 2 Substituenten unabhängig voneinander ausgewählt aus der Gruppe Fluor, (C₁-C₄)-Alkyl, Hydroxy und Amino substituiert sein kann,
und
wobei (C₁-C₃)-Alkandlyl mit 1 Substituenten ausgewählt aus der Gruppe Fluor und Trifluormethyl substituiert ist,
R¹ für Wasserstoff oder Fluor steht,
R² für Benzyl steht,
wobei Benzyl mit 1 bis 3 Substituenten Fluor substituiert ist,
sowie ihre Salze, Solvate und Solvate der Salze.

4. Verfahren zur Herstellung von Verbindungen der Formel (I), wie in den Ansprüchen 1 bis 3 definiert, **dadurch gekennzeichnet, dass** man
[A] eine Verbindung der Formel (II) 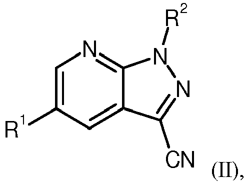 in welcher R¹ und R² jeweils die in den Ansprüchen 1 bis 3 angegebenen Bedeutungen haben,
unter sauren Bedingungen in eine Verbindung der Formel (III) 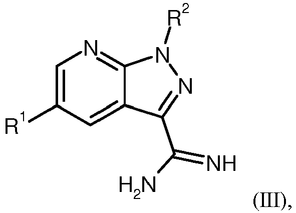 in welcher R¹ und R² jeweils die in den Ansprüchen 1 bis 3 angegebenen Bedeutungen haben,
überführt, diese im Folgenden in einem inerten Lösungsmittel in Gegenwart einer geeigneten Base mit einer Verbindung der Formel (IV) 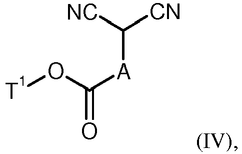 in welcher A die in den Ansprüchen 1 bis 3 angegebene Bedeutung hat und
T¹ für (C₁-C₄)-Alkyl steht,
zu einer Verbindung der Formel (I) 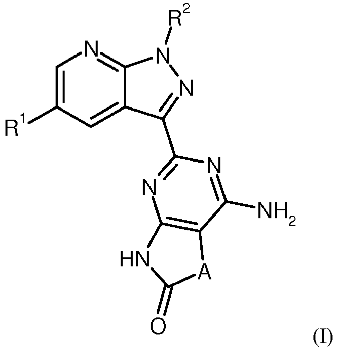 in welcher A, R¹ und R² jeweils die in den Ansprüchen 1 bis 3 angegebenen Bedeutungen haben,
umsetzt
oder
[B] eine Verbindung der Formel (III) in einem inerten Lösungsmittel in Gegenwart einer geeigneten Base mit einer Verbindung der Formel (V) 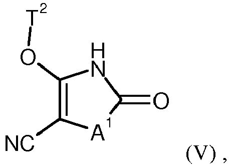 in welcher
A¹ für (C₂-C₃)-Alkandiyl steht,
wobei (C₂-C₃)-Alkandiyl mit 1 oder 2 Substituenten unabhängig voneinander ausgewählt aus der Gruppe Fluor, Trifluormethyl und (C₁-C₄)-Alkyl substituiert sein kann,
worin (C₁-C₄)-Alkyl mit 1 bis 3 Substituenten unabhängig voneinander ausgewählt aus der Gruppe Fluor, Trifluormethyl und Hydroxy substituiert sein kann,
und
T² für (C₁-C₄)-Alkyl steht,
zu einer Verbindung der Formel (I-A) 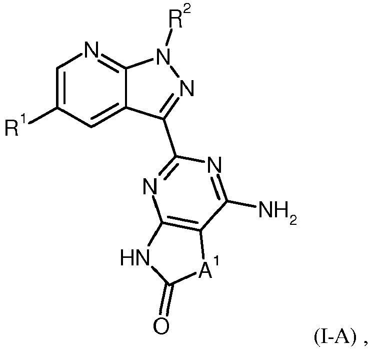 in welcher A¹, R¹ und R² jeweils die in den Ansprüchen 1 bis 3 angegebenen Bedeutungen haben,
umsetzt,
und die resultierenden Verbindungen der Formeln (I) und (I-A) gegebenenfalls mit den entsprechenden (i) Lösungsmitteln und/oder (ii) Säuren oder Basen in ihre Solvate, Salze und/oder Solvate der Salze überführt.

5. Verbindung der Formel (I), wie in einem der Ansprüche 1 bis 3 definiert, zur Verwendung in der Behandlung und/oder Prophylaxe von Krankheiten.

6. Verwendung einer Verbindung der Formel (I), wie in einem der Ansprüche 1 bis 3 definiert, zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

7. Verbindung der Formel (I) zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

8. Arzneimittel enthaltend eine Verbindung der Formel (I), wie in einem der Ansprüche 1 bis 3 definiert, in Kombination mit einem inerten, nicht-toxischen, pharmazeutisch geeigneten Hilfsstoff.

9. Arzneimittel enthaltend eine Verbindung der Formel (I), wie in einem der Ansprüche 1 bis 3 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

10. Arzneimittel nach Anspruch 8 oder 9 zur Verwendung in der Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

## Claims

1. Compound of the formula (I) 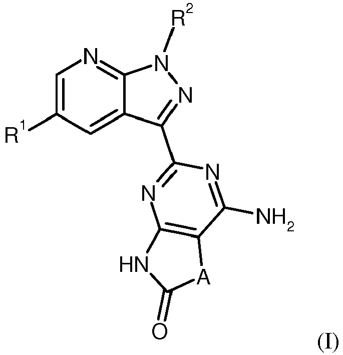 in which
A is (C₁-C₃)-alkanediyl or a group of the formula 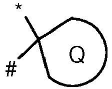 where
* is the attachment site to the pyrimidine ring,
# is the attachment site to the carbonyl group,
the ring Q is a 4- to 6-membered heterocycle,
and
where (C₁-C₃)-alkanediyl may be substituted by 1 or 2 substituents independently selected from the group of fluorine, trifluoromethyl, (C₁-C₄)-alkyl, hydroxyl and amino,
in which (C₁-C₄)-alkyl may be substituted by 1 to 3 substituents independently selected from the group of fluorine, trifluoromethyl and hydroxyl,
R¹ is hydrogen or fluorine,
R² is benzyl,
where benzyl is substituted by 1 to 3 fluorine substituents, and the *N*-oxides, salts, solvates, salts of the *N*-oxides and solvates of the *N-*oxides or salts thereof.

2. Compound of the formula (I) according to Claim 1, in which
A is a group of the formula 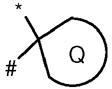 where
* is the attachment site to the pyrimidine ring,
# is the attachment site to the carbonyl group,
the ring Q is an azetidinyl, oxetanyl, pyrrolidinyl, tetrahydrofuranyl, piperidinyl or tetrahydropyranyl ring,
R¹ is hydrogen or fluorine,
R² is benzyl,
where benzyl is substituted by 1 to 3 fluorine substituents, and the salts, solvates and solvates of the salts thereof.

3. Compound of the formula (I) according to Claim 1, in which
A is (C₁-C₃)-alkanediyl
where (C₁-C₃)-alkanediyl may be substituted by 1 or 2 substituents independently selected from the group of fluorine, (C₁-C₄)-alkyl, hydroxyl and amino,
and
where (C₁-C₃)-alkanediyl is substituted by 1 substituent selected from the group of fluorine and trifluoromethyl,
R¹ is hydrogen or fluorine,
R² is benzyl,
where benzyl is substituted by 1 to 3 fluorine substituents,
and the salts, solvates and solvates of the salts thereof.

4. Process for preparing compounds of the formula (I) as defined in Claims 1 to 3, **characterized in that**
[A] a compound of the formula (II) 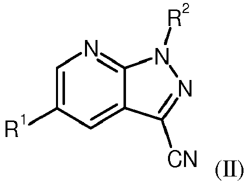 in which R¹ and R²- are each as defined in Claims 1 to 3,
is converted under acidic conditions to a compound of the formula (III) 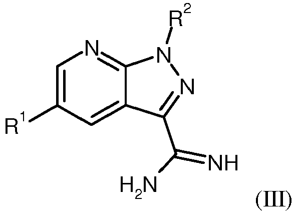 in which R¹ and R²- are each as defined in Claims 1 to 3,
the latter is reacted in an inert solvent in the presence of a suitable base with a compound of the formula (IV) 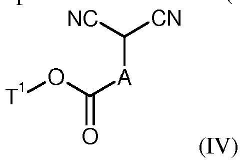 in which A is as defined in Claims 1 to 3 and
T¹ is (C₁-C₄)-alkyl,
to give a compound of the formula (I) 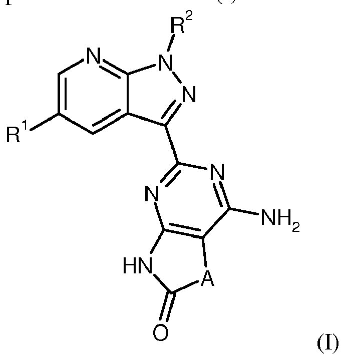 in which A, R¹ and R² are each as defined in Claims 1 to 3,
or
[B] a compound of the formula (III) is reacted in an inert solvent in the presence of a suitable base with a compound of the formula (V) 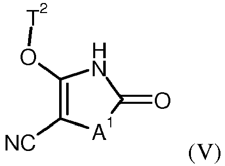 in which
A¹ is (C₂-C₃)-alkanediyl,
where (C₂-C₃)-alkanediyl may be substituted by 1 or 2 substituents independently selected from the group of fluorine, trifluoromethyl and (C₁-C₄)-alkyl,
in which (C₁-C₄)-alkyl may be substituted by 1 to 3 substituents independently selected from the group of fluorine, trifluoromethyl and hydroxyl,
and
T² is (C₁-C₄)-alkyl,
to give a compound of the formula (I-A) 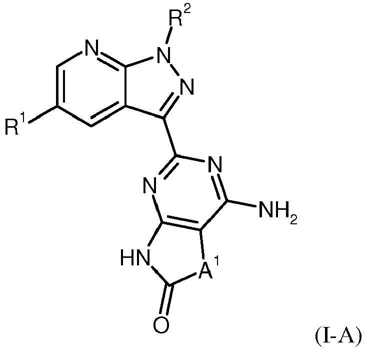 in which A¹, R¹ and R²- are each as defined in Claims 1 to 3,
and the resulting compounds of the formulae (I) and (I-A) are optionally converted with the appropriate (i) solvents and/or (ii) acids or bases to the solvates, salts and/or solvates of the salts thereof.

5. Compound of the formula (I) as defined in any of Claims 1 to 3 for use in the treatment and/or prophylaxis of diseases.

6. Use of a compound of the formula (I) as defined in any of Claims 1 to 3 for production of a medicament for treatment and/or prophylaxis of heart failure, angina pectoris, hypertension, pulmonary hypertension, ischemia, vascular disorders, kidney failure, thromboembolic disorders, fibrotic disorders and arteriosclerosis.

7. Compound of the formula (I) for use in a method for treatment and/or prophylaxis of heart failure, angina pectoris, hypertension, pulmonary hypertension, ischemia, vascular disorders, kidney failure, thromboembolic disorders, fibrotic disorders and arteriosclerosis.

8. Medicament comprising a compound of the formula (I) as defined in any of Claims 1 to 3 in combination with an inert, nontoxic, pharmaceutically suitable excipient.

9. Medicament comprising a compound of the formula (I) as defined in any of Claims 1 to 3 in combination with a further active ingredient selected from the group consisting of organic nitrates, NO donors, cGMP-PDE inhibitors, antithrombotic agents, hypotensive agents and lipid metabolism modifiers.

10. Medicament according to Claim 8 or 9 for use in the treatment and/or prophylaxis of heart failure, angina pectoris, hypertension, pulmonary hypertension, ischemia, vascular disorders, kidney failure, thromboembolic disorders, fibrotic disorders and arteriosclerosis.

## Revendications

1. Composé de formule (I) 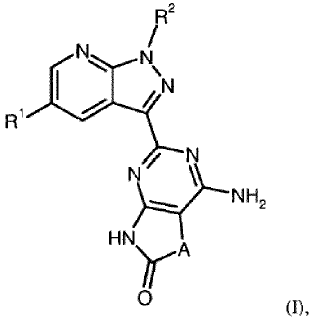 dans laquelle
A représente un groupe alcane(C₁-C₃)diyle ou un groupe de formule 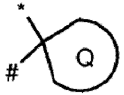 où
* désigne le point d'attachement au cycle pyrimidine,
# désigne le point d'attachement au groupe carbonyle,
le cycle Q représente un hétérocycle à 4 à 6 chaînons,
et
le groupe alcane(C₁-C₃)diyle pouvant être substitué par 1 ou 2 substituants choisis indépendamment l'un de l'autre dans le groupe constitué par fluoro, trifluorométhyle, alkyle en C₁-C₄, hydroxy et amino,
le substituant alkyle en C₁-C₄ pouvant être substitué par 1 à 3 substituants choisis chacun indépendamment dans le groupe constitué par fluoro, trifluorométhyle et hydroxy,
R¹ représente un atome d'hydrogène ou de fluor,
R² représente un groupe benzyle,
le groupe benzyle étant substitué par 1 à 3 substituants fluoro,
ainsi que ses *N*-oxydes, sels, produits de solvatation, sels des *N*-oxydes et produits de solvatation des *N*-oxydes et sels.

2. Composé de formule (I) selon la revendication 1, dans lequel
A représente un groupe de formule 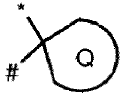 où
* désigne le point d'attachement au cycle pyrimidine,
# désigne le point d'attachement au groupe carbonyle,
le cycle Q représente un cycle azétidinyle, oxétanyle, pyrrolidinyle, tétrahydrofuranyle, pipéridinyle ou tétrahydropyranyle,
R¹ représente un atome d'hydrogène ou de fluor,
R² représente un groupe benzyle,
le groupe benzyle étant substitué par 1 à 3 substituants fluoro,
ainsi que ses sels, produits de solvatation et produits de solvatation des sels.

3. Composé de formule (I) selon la revendication 1, dans lequel
A représente un groupe alcane(C₁-C₃)diyle
le groupe alcane(C₁-C₃)diyle pouvant être substitué par 1 ou 2 substituants choisis indépendamment l'un de l'autre dans le groupe constitué par fluoro, alkyle en C₁-C₄, hydroxy et amino,
et
le groupe alcane(C₁-C₃)diyle étant substitué par 1 substituant choisi dans le groupe constitué par fluoro et trifluorométhyle,
R¹ représente un atome d'hydrogène ou de fluor,
R² représente un groupe benzyle,
le groupe benzyle étant substitué par 1 à 3 substituants fluoro,
ainsi que ses sels, produits de solvatation et produits de solvatation des sels.

4. Procédé pour la préparation des composés de formule (I), tels que définis dans les revendications 1 à 3, **caractérisé en ce que**
[A] on convertit un composé de formule (II) 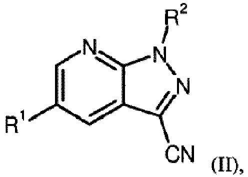 dans laquelle R¹ et R² ont chacun les significations indiquées dans les revendications 1 à 3,
dans des conditions acides, en un composé de formule (III) 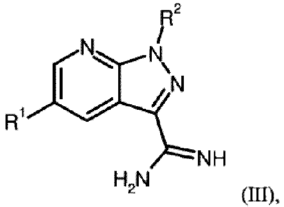 dans laquelle R¹ et R² ont chacun les significations indiquées dans les revendications 1 à 3,
ensuite on fait réagir ce dernier, dans un solvant inerte, en présence d'une base convenable, avec un composé de formule (IV) 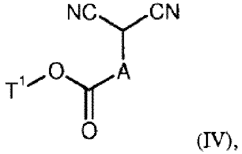 dans laquelle A a la signification indiquée dans les revendications 1 à 3 et
T¹ représente un groupe alkyle en C₁-C₄,
pour aboutir à un composé de formule (I) 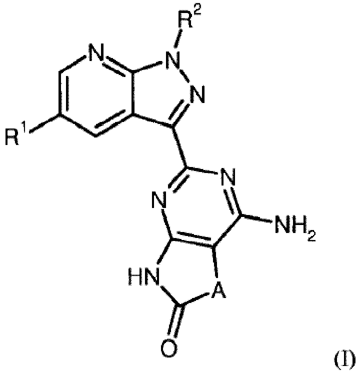 dans laquelle A, R¹ et R² ont chacun les significations indiquées dans les revendications 1 à 3,
ou
[B] on fait réagir un composé de formule (III), dans un solvant inerte, en présence d'une base convenable, avec un composé de formule (V) 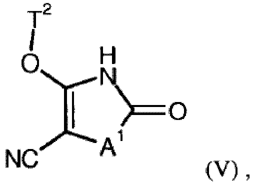 dans laquelle
A¹ représente un groupe alcane(C₂-C₃)diyle,
le groupe alcane (C₂-C₃) diyle pouvant être substitué par 1 ou 2 substituants choisis indépendamment l'un de l'autre dans le groupe constitué par fluoro, trifluorométhyle et alkyle en C₁-C₄,
le substituant alkyle en C₁-C₄ pouvant être substitué par 1 à 3 substituants choisis chacun indépendamment dans le groupe constitué par fluoro, trifluorométhyle et hydroxy,
et
T² représente un groupe alkyle en C₁-C₄,
pour aboutir à un composé de formule (I-A) 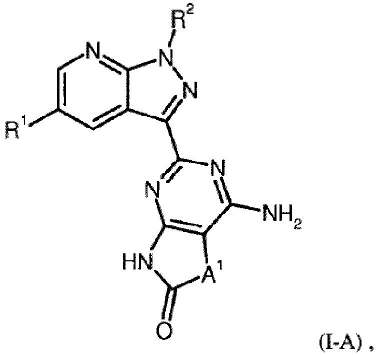 dans laquelle A¹, R¹ et R² ont chacun les significations indiquées dans les revendications 1 à 3,
et éventuellement on convertit les composés de formules (I) et (I-A) résultants, à l'aide (i) des solvants correspondants et/ou (ii) des bases correspondantes ou des acides correspondants, en leurs produits de solvatation, sels et/ou produits de solvatation des sels.

5. Composé de formule (I), tel que défini dans l'une quelconque des revendications 1 à 3, pour l'utilisation dans le traitement et/ou la prophylaxie de maladies.

6. Utilisation d'un composé de formule (I), tel que défini dans l'une quelconque des revendications 1 à 3, pour la fabrication d'un médicament destiné au traitement et/ou à la prophylaxie de l'insuffisance cardiaque, de l'angine de poitrine, de l'hypertonie, de l'hypertonie pulmonaire, d'ischémies, de maladies vasculaires, de l'insuffisance rénale, de maladies thromboemboliques, de maladies fibreuses et de l'artériosclérose.

7. Composé de formule (I) pour l'utilisation dans un procédé destiné au traitement et/ou à la prophylaxie de l'insuffisance cardiaque, de l'angine de poitrine, de l'hypertonie, de l'hypertonie pulmonaire, d'ischémies, de maladies vasculaires, de l'insuffisance rénale, de maladies thromboemboliques, de maladies fibreuses et de l'artériosclérose.

8. Médicament contenant un composé de formule (I), tel que défini dans l'une quelconque des revendications 1 à 3, en association avec un adjuvant inerte, non toxique, pharmaceutiquement convenable.

9. Médicament contenant un composé de formule (I), tel que défini dans l'une quelconque des revendications 1 à 3, en association avec une autre substance active choisie dans le groupe constitué par des nitrates organiques, des donneurs de NO, des inhibiteurs de GMPc-PDE, des agents à action antithrombotique, des agents abaissant la tension artérielle ainsi que des agents modifiant le métabolisme lipidique.

10. Médicament selon la revendication 8 ou 9, destiné à l'utilisation dans le traitement et/ou la prophylaxie de l'insuffisance cardiaque, de l'angine de poitrine, de l'hypertonie, de l'hypertonie pulmonaire, d'ischémies, de maladies vasculaires, de l'insuffisance rénale, de maladies thromboemboliques, de maladies fibreuses et de l'artériosclérose.
